(19) Europäisches Patentamt
European Patent Office
Office européen des brevets

(11) **EP 2 875 343 B1**

(12) **EUROPEAN PATENT SPECIFICATION**

(45) Date of publication and mention of the grant of the patent:
**14.03.2018   Bulletin 2018/11**

(51) Int Cl.:
*G01N 27/403* *(2006.01)*     *G01N 27/30* *(2006.01)*
*C12Q 1/04* *(2006.01)*     *G01N 27/00* *(2006.01)*
*B82Y 30/00* *(2011.01)*

(21) Application number: **13819564.9**

(22) Date of filing: **22.07.2013**

(86) International application number:
**PCT/US2013/051471**

(87) International publication number:
**WO 2014/015333 (23.01.2014 Gazette 2014/04)**

(54) **MICROFLUIDIC-NANOFLUIDIC DEVICES FOR DETECTION AND MEASUREMENT OF REDOX ACTIVE SUBSTANCES**

MIKROFLUIDISCH-NANOFLUIDISCHE VORRICHTUNGEN ZUR DETEKTION UND MESSUNG REDOX-AKTIVER SUBSTANZEN

DISPOSITIFS MICROFLUIDIQUES ET NANOFLUIDIQUES POUR LA DÉTECTION ET LA MESURE DE SUBSTANCES À ACTIVITÉ REDOX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **20.07.2012   US 201261673852 P**

(43) Date of publication of application:
**27.05.2015   Bulletin 2015/22**

(73) Proprietor: **Northeastern University Boston, MA 02115 (US)**

(72) Inventors:
• **GOLUCH, Edgar, D.**
  **Somerville, MA 02144 (US)**
• **WEBSTER, Thaddaeus**
  **Exeter, New Hampshire 03833 (US)**

(74) Representative: **Høiberg P/S Adelgade 12 1304 Copenhagen K (DK)**

(56) References cited:
WO-A2-2004/105153     US-A1- 2007 208 243
US-A1- 2011 155 586     US-A1- 2011 227 558
US-A1- 2011 266 151     US-B2- 8 202 408

• Webster T A: "Detection of quorum sensing molecules in nanofluidic detection devices", NORTHEASTERN UNIVERSITY, 23 July 2011 (2011-07-23), XP055182376, Retrieved from the Internet: URL:http://nuweb9.neu.edu/chegsc/wp-conten t/uploads/Webster-Abstract-03-11-111.pdf [retrieved on 2015-04-13]
• ODIJK M ET AL: "A microfluidic chip for electrochemical conversions in drug metabolism studies", LAB ON A CHIP, vol. 9, no. 12, 23 March 2009 (2009-03-23) , pages 1687-1693, XP055081559, ISSN: 1473-0197, DOI: 10.1039/b822962g
• GOLUCH E D ET AL: "Redox cycling in nanofluidic channels using interdigitated electrodes", ANALYTICAL AND BIOANALYTICAL CHEMISTRY, vol. 394, no. 2, 6 January 2009 (2009-01-06), pages 447-456, XP019702599, ISSN: 1618-2650
• ZHOU J ET AL: "Fabrication of a microfluidic Ag/AgCl reference electrode and its application for portable and disposable electrochemical microchips", ELECTROPHORESIS, vol. 31, no. 18, 30 August 2010 (2010-08-30), pages 3083-3089, XP055259181, DE ISSN: 0173-0835, DOI: 10.1002/elps.201000113

- SHARP D ET AL: "Approaching intelligent infection diagnostics: Carbon fibre sensor for electrochemical pyocyanin detection", BIOELECTROCHEMISTRY, vol. 77, no. 2, 22 July 2009 (2009-07-22), pages 114-119, XP026870639, ISSN: 1567-5394 [retrieved on 2009-07-22]
- WEBSTER T A ET AL: "Electrochemical detection of pyocyanin in nanochannels with integrated palladium hydride reference electrodes", LAB ON A CHIP, vol. 12, no. 24, 10 October 2012 (2012-10-10), pages 5195-5201, XP055257916, GB ISSN: 1473-0197, DOI: 10.1039/c2lc40650k
- WEBSTER, T.: 'Detection of Quorum Sensing Molecules in Nanofluidic Detection Devices.' NORTHEASTERN UNIVERSITY. 23 July 2011, XP055182376 Retrieved from the Internet: <URL:http://nuweb9.neu.edu/chegsGwp-contenU uploads/Webster-Abstract-03-11-111.pdf> [retrieved on 2013-11-22]

## Description

The disclosure was developed with financial support from Grant No. 1125535 from the National Science Foundation. The U.S. Government has certain rights in the disclosure.

## BACKGROUND

Sensitive and selective detection of redox active substances, including those produced by pathogenic microorganisms, has important implications for both medical and environmental research, and in microbial fuel cells (Zhang et al., 2005, Curr Opin Microbial 8, 276-281; Jacob et al., 2011, Current Opinion in Chem Biol 15, 149-155; Dietrich et al., 2008, Science 321, 1203-1206; and Ren et al., 2012, Microfluid Nanofluid 13, 353-381). Further, the growing rate of hospital infections and the emergence of antibiotic resistant bacteria, such as antibiotic resistant strains of *Pseudomonas aeruginosa* (PA) highlight the need for small, robust sensors that detect bacterial contamination. (Paramythiotou et al.,2004, Clin Infect Dis 38, 670-677). A variety of amperometric and potentiometric techniques can be utilized to obtain information about the chemical composition of a solution (Pihel et al., 1995 Anal Chem, 67, 4514-4521; Buck et al., 2001, Anal Chem, 73, 88-97; Hengstenberg et al., 2001, Angew Chem Int Ed Engl, 40, 905-908; Müller et al., 1981, J Neuro Meth, 1981, 4, 39-52), and may be used for development of the sensors for detecting redox active substances.

Electrochemical detection offers several advantages over other sensing schemes, such as fast analysis time, ease of use, and low limits of detection (Cheng et al., 2007, Electrophoresis 28, 1579-1586; Zou et al., 2008, IEEE Sens J 8, 527-535; Zevenbergen et al., 2007, Nano Lett 7, 384-388 ). Low-fabrication cost microscale electrochemical systems which have smaller sample volume requirements are attractive for the detection of molecules by this method.(Ino et al., 2011, Lab Chip 11, 385-388; Hwang et al., 2009, IEEE Sens J 9, 609-615).

Each of the amperometric and potentiometric techniques requires a stable reference electrode to provide accurate measurements. With the emergence of micro fabrication techniques, miniaturized electrochemical sensors are now being developed and integrated inside fluidic systems (Lewis et al., 2010, Anal Chem, 82, 1659-1668; Kwakye et al., 2006 Biosens Bioelectron, 21, 2217-2223; Swensen, et al., 2009, J Am Chem Soc,131, 4262-4266; Wang et al., 2008, Sensors, 8, 2043-2081; Straver, et al., 2012, Lab Chip, 12, 1548-1553). However, many of these systems rely on macroscale reference electrodes that are not directly inserted into the microfluidic environment or pseudo reference electrodes that are not stable over multiple days (Dam et al.,2007 Analyst, 132, 365-370; Niwa et al., 1991, Electroanal, 3, 163-168; Goluch et al., 2009, Anal Bioanal Chem, 394, 447-456; Kätelhön et al., 2010, Anal Chem, 82, 8502-8509).

Thus, there remains a need for electrochemical sensors having reliable and stable miniaturized reference electrodes.

The abstract Webster T A: "Detection of quorum sensing molecules in nanofluidic detection devices", NORTHEASTERN UNIVERSITY, 23 July 2011 (2011-07-23), XP055182376 describes the fabrication and use of nanofluidic electrochemical sensors composed of two working electrodes, spaced only a few nanometers apart, which have a potential applied between them. As electrochemical molecules pass between the two electrodes a current is generated due to redox cycling. The sensors are constructed on silicon dioxide (SiO2) wafers. First gold is layered onto the wafer followed by sacrificial chromium layer. On top of this another gold layer is added and in turn is encased by SiO2. Holes are made in the SiO2 down to the chromium layer using reactive ion etching. The chromium layer is then dissolved with chromium etchant leaving behind a nanofluidic chamber, with the two gold layers acting as electrodes. The sensors are used to detect the quorum sensing molecule pyocyanin being emitted from single E. coli cells.

US 2011/155586 A1 describes an electrochemical sensor having at least two electrodes separated by a nanoscale gap wherein the separation between the first electrode and the second electrode forms a cavity capable of containing a fluid. Optionally, the device includes a reference electrode and is capable of detecting chemicals and biochemicals through redox cycling events. Embodiments are adapted to identify and sequence nucleic acid molecules.

## SUMMARY OF THE DISCLOSURE

Devices for electrochemical measurement of a concentration of a redox active substance, methods of fabricating the devices, and methods of measuring a concentration of a redox active substance using the devices are provided.

As used herein, the term "nanoscale" refers to an object or a feature whose size is in the range from about 1 nm to about 999 nm, or less than 1 $\mu$m. The term "microscale" refers to an object or feature whose size is in the range from about 1 $\mu$m to about 999 $\mu$m, or less than 1 mm. A "nanofluidic channel" as used herein is a space generally in the form of a channel and having a cross-sectional dimension of nanoscale size. Other dimensions of a nanofluidic channel, such as its length can be of microscale size or greater, or can also be of nanoscale size. A "microfluidic channel" as used herein is a channel having all cross-sectional dimensions in the microscale range or greater.

An embodiment according to the present disclosure is a device for measurement of a concentration of a redox active substance in a sample, the device including: a nanofluidic electrode assembly having a nanofluidic channel disposed in a substrate, a working electrode disposed in the nanofluidic channel, and a reference electrode disposed in the nanofluidic channel; a microfluidic channel in said substrate, the microfluidic channel in fluid communication with the nanofluidic channel; such that the working electrode is suitable for applying a potential sufficient to alternately oxidize and reduce the redox active substance; and such that the concentration of the redox active substance present in the nanofluidic channel is measured as a current flow through the working electrode. In related embodiments according to the present disclosure, the device further includes a counter electrode. For example, the counter electrode monitors the current flowing through the reference electrode and is useful to keep the current flow through the reference electrode to a low level, such as less than about 1nA.

Another embodiment according to the present disclosure is a device for measurement of a concentration of a redox active substance in a sample, the device including: a nanofluidic electrode assembly having a nanofluidic channel disposed in a substrate, an oxidizing working electrode disposed in the nanofluidic channel, a reducing working electrode disposed in the nanofluidic channel, and a reference electrode disposed in the nanofluidic channel; a microfluidic channel disposed in said substrate, the microfluidic channel in fluid communication with the nanofluidic channel; such that the oxidizing working electrode is suitable for applying a potential sufficient to oxidize the redox active substance and the reducing working electrode is suitable for applying a potential sufficient to reduce the redox active substance; such that the concentration of the redox active substance present in the nanofluidic channel is measured as a current flow through the working electrode(s). In related embodiments according to the present disclosure, the oxidizing and the reducing working electrodes are separated by a distance of about 20 - 100 nm across the lumen of the nanofluidic channel.

In other related embodiments according to the present disclosure, the substrate of the devices includes a glass having a refractive index greater than 1.5. For example, the refractive index is in the range of 1.6-1.9. For example, the glass has a thickness of about 100 nm.

Related embodiments according to the present disclosure include devices that further include integrated Complementary Metal-Oxide Semiconductor (CMOS) electronics and a wireless transmitter.

The devices, in related embodiments according to the present disclosure, are suitable for measurement of a concentration of a redox active substance in a sample produced by a microorganism. In other related embodiments according to the present disclosure, the redox active substance is pyocyanin produced by *Pseudomonas aeruginosa*.

In various embodiments according to the present disclosure, the working electrode(s) of the devices include a material selected from the group consisting of: gold, electrically conductive diamond, platinum, and glassy carbon. In related embodiments according to the present disclosure, the reference electrode includes palladium.

Embodiments according to the present disclosure include devices that are configured for use as a medical device or a component of a medical device. In related embodiments according to the present disclosure, devices are disposable. In other related embodiments according to the present disclosure, a device is configured for implantation in a patient.

According to another embodiment according to the present disclosure, the reference electrode of the device forms at least a portion of a wall of the nanochannel. In related embodiments according to the present disclosure, a working electrode in the device forms at least a portion of a wall of the nanochannel.

In related embodiments according to the present disclosure, in the device having one working electrode, the reference electrode and the working electrode each form at least a portion of a wall of the nanochannel, and are separated by an insulating portion of the nanochannel, the insulating portion extending from about 30 nm to about 50 $\mu$m along the length of the nanochannel.

Related embodiments according to the present disclosure include devices having an oxidizing and a reducing working electrode such that the oxidizing and reducing working electrodes form portions of opposite facing walls of the nanochannel, the opposite facing walls separated by a distance of about 20-100 nm. In other related embodiments according to the present disclosure the reference electrode and the working electrodes each form at least a portion of a wall of the nanochannel, and the reference electrode is separated from both working electrodes by an insulating portion of the nanochannel, the insulating portion extending from about 30 nm to about 50 $\mu$m along the length of the nanochannel.

In some related embodiments according to the present disclosure, the working electrode(s) have a greater surface area than that of the reference electrode.

Embodiments according to the present disclosure include a device having a working and a reference electrode, such that the working electrode has a surface area of at least 100 $nm^2$. In related embodiments according to the present disclosure, the area of the working electrode is less than 100000, less than 50000, less than 20000, less than 10000, or less than 500 $nm^2$.

In other embodiments according to the present disclosure, the device has an oxidizing working electrode and a reducing working electrode, and the working electrodes each have a surface area of at least 1 $\mu m^2$. In related embodiments according to the present disclosure, the area of each working electrode is less than 100, less than 50, less than 20, less than 10, or less than 5 $\mu m^2$.

In related embodiments according to the present disclosure the one or the two working electrode devices above are such that the volume of the nanochannel in which the electrodes are disposed is less than about 50 nanoliters (nL), less than about 10 nL, less than about 1 nL, less than about 100 picoliters (pL), less than about 50 pL less than about 10 pL, less than about 5 pL, or less than about 1 pL.

Related embodiments according to the present disclosure include devices such that the width of the nanochannel in which the electrodes are disposed is less than 20 $\mu$m. In other related embodiments according to the present disclosure, the device further includes inlet and outlet ports in fluid connection with the microchannel, the ports configured to add and remove the sample to and from the microchannel. Related embodiments according to the present disclosure also include a device further including a processor that performs data analysis using the current flow through the working electrode(s). Also included as related embodiments according to the present disclosure are devices that display the current flow through the working electrode(s), or display the concentration of the redox active substance.

Another related embodiment according to the present disclosure is a device for simultaneous measurement of a concentration of each of a plurality of redox active substances in a sample, the device comprising a plurality of devices according to embodiments above according to the present disclosure, having a working electrode and a reference electrode.

Included as yet another related embodiment according to the present disclosure is a device for simultaneous measurement of a concentration of each of a plurality of redox active substances in a sample, the device comprising a plurality of devices according to the embodiments above according to the present disclosure having an oxidizing working electrode, a reducing working electrode, and a reference electrode.

In related embodiments according to the present disclosure is a device for simultaneous measurement of a concentration of a redox active substance, and a presence of a biomass, the device including either the one working electrode or the two working electrodes embodiments above according to the present disclosure, glass having a refractive index greater than 1.5 as the substrate, and further including a window for measuring surface plasmon resonance of one of the working electrodes, such that the surface plasmon resonance indicates the presence of the biomass at said working electrode. In related embodiments according to the present disclosure, the disclosure is a device for simultaneous measurement of an amount of each of a plurality of redox active substances, and each of a plurality of biomass, the device comprising a plurality of devices, each including either the one working electrode or the two working electrodes embodiments above according to the present disclosure, glass having a refractive index greater than 1.5 as the substrate, and further including a window for measuring surface plasmon resonance of one of the working electrodes, such that the surface plasmon resonance indicates the presence of the biomass at said working electrode.

Another embodiment according to the present disclosure is a method of fabricating a nanoscale device for measurement of a concentration of a redox active substance in a sample, the device including a nanofluidic electrode assembly in fluid communication with a microfluidic channel, the method including the steps of: depositing a first electrode layer on a substrate; depositing a sacrificial layer on the first electrode layer; depositing a reference electrode layer on the sacrificial layer; depositing an insulating layer on the reference electrode layer; making two or more holes through the insulating layer, the holes providing a fluid connection to the sacrificial layer; applying an etching agent through the holes, whereby the sacrificial layer is etched away to form a nanochannel, the first electrode layer and reference electrode layer each forming a portion of a wall of the nanochannel; and preparing a microfluidic channel in the substrate, the microfluidic channel in fluid connection with the nanochannel. In related embodiments according to the present disclosure the method further includes applying a resist material over one or more layers and performing lithography to create a patterned mask prior to applying a subsequent layer. Another related embodiment according to the present disclosure of the method includes bonding a wire to an electrode layer. In various embodiments according to the present disclosure the microfluidic channels have forms selected from the group of: a channel, a reservoir, an open access port, and an open access port covered with a semipermeable membrane.

According to other related embodiments according to the present disclosure, in the method for fabricating a nanoscale device the thickness of the first electrode layer is about 20 nm. In other embodiments according to the present disclosure the thickness of the reference electrode layer is about 120 nm. Related embodiments according to the present disclosure include a method wherein the thickness of the sacrificial layer is about 20-200 nm. Further, in other related embodiments according to the present disclosure, the thickness of the insulating layer is about 500 nm. Embodiments according to the present disclosure include those in which the insulating layer includes silicon dioxide. Embodiments according to the present disclosure also include those in which the insulating layer includes a first layer of oxide, a layer of nitride, and a second layer of oxide. For example, the first and the second layer of oxide include $SiO_2$, and the layer of nitride includes $Si_3N_4$.

In various related embodiments according to the present disclosure, the method is such that the first electrode layer includes a material selected from the group consisting of: gold, electrically conductive diamond, platinum, and glassy carbon. In other related embodiments according to the present disclosure the reference electrode layer includes palladium. In some embodiments according to the present disclosure the electrode layers are deposited by a method selected from the group consisting of: electron beam deposition, physical vapor deposition, and chemical vapor deposition. In related embodiments according to the present disclosure the method further includes depositing a second electrode layer on the sacrificial layer, the second layer and the reference layer being essentially coplanar and non-adjacent. In various embodiments according to the present disclosure the substrate is a silicon wafer having a surface layer of $SiO_2$.

Embodiments according to the present disclosure include a method of measuring a concentration of a redox active substance present in a sample liquid including: providing a device having a one working electrode, or two working electrodes, as described in embodiments above according to the present disclosure, including the sample liquid loaded into the nanofluidic channel of the device; (ii) charging the reference electrode to stabilize its potential; (ii) recording the current flow through the working electrode(s); and (iv) determining a concentration of the substance producing the current flow by using a previously determined correlation between known concentrations of the substance and the current flow through the working electrode(s), recorded according to steps (i) -(iii). In related embodiments according to the present disclosure the device includes both oxidizing and reducing working electrodes, the method including: applying a potential suitable for oxidizing the substance to the oxidizing electrode, and a potential suitable for reducing the substance to the reducing electrode; and such that the substance has a half way potential value between the potential of the oxidizing electrode and the potential of the reducing electrode. For example, the sample is a bodily fluid selected from the group consisting of: bronchial lavage, sputum, urine, saliva, spinal fluid, and blood.

According to various related embodiments according to the present disclosure, the method further includes determining pH of the sample using a pH electrode disposed within the nanochannel, and a previously determined correlation between known amounts of the substance, each in a medium of known pH, and currents produced by the known amounts of the substance, recorded according to the steps (i) -(iii). In other related embodiments according to the present disclosure, the redox active substance is produced by a microorganism, the method further including: determining the effect of an agent on the microorganism by measuring amounts of the redox active substance produced by the microorganism upon treating the microorganism with defined amounts of the agent. For example, the agent is selected from the group consisting of an antibiotic, an antibody, an siRNA, a nanoparticle, and a signaling molecule.

Another embodiment according to the present disclosure is a method of measuring a concentration of a redox active substance present in a sample and a presence of a biomass including: (i) providing a device for simultaneous measurement of a concentration of a redox active substance and a presence of a biomass as described above, including the sample loaded into the microfluidic channel of the device; (ii) charging the reference electrode to stabilize its potential; (ii) recording the current flow through the working electrode(s); and (iv) determining a concentration of the substance producing the current flow by using a previously determined correlation between known concentrations of the substance and the current flow through the working electrode(s), recorded according to steps (i) -(iii), and (v) determining the presence of the biomass by measuring surface plasmon resonance changes at the working electrode proximal to the substrate. For example, the biomass is selected from the group consisting of: microorganisms in a medium, aggregates of microorganisms in a medium, and a biofilm of microorganisms. In related embodiments according to the present disclosure the working electrode is functionalized with a nucleic acid, a peptide, or an antibody.

BRIEF DESCRIPTION OF THE DRAWINGS

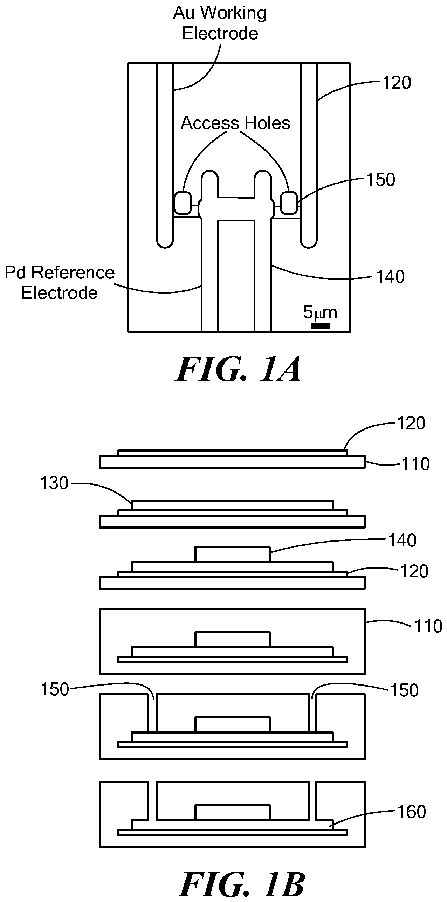

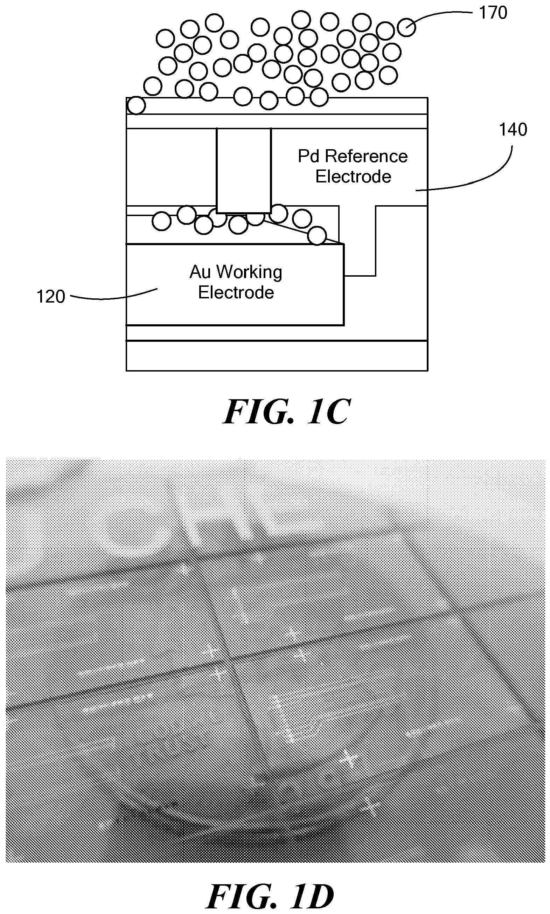

FIGS. 1 A-D are an optical micrograph and schematic diagrams of a completed device having a nanofluidic electrode assembly. FIG. 1A is a top view optical micrograph of a complete device after removal of the sacrificial chromium (Cr) layer. FIG. 1B is a side view of the device fabrication scheme showing (1) deposition of 20 nm gold working electrode onto the surface of a $SiO_2$ wafer; (2) deposition of 60 nm sacrificial Cr layer; (3) deposition of 120 nm palladium (Pd) hydride reference electrode; (4) 550 nm of $SiO_2$ sputtered onto metals, and access holes etched down to Cr layer; and (5) removal of Cr layer with Cr etchant. FIG. 1C is a perspective view schematic of a complete device with redox active molecules (spheres; not to scale). FIG. 1D is a photograph of a nano-electrochemical sensor having a nanoelectrode assembly with a single working electrode and a Pd reference electrode fabricated on a glass wafer.

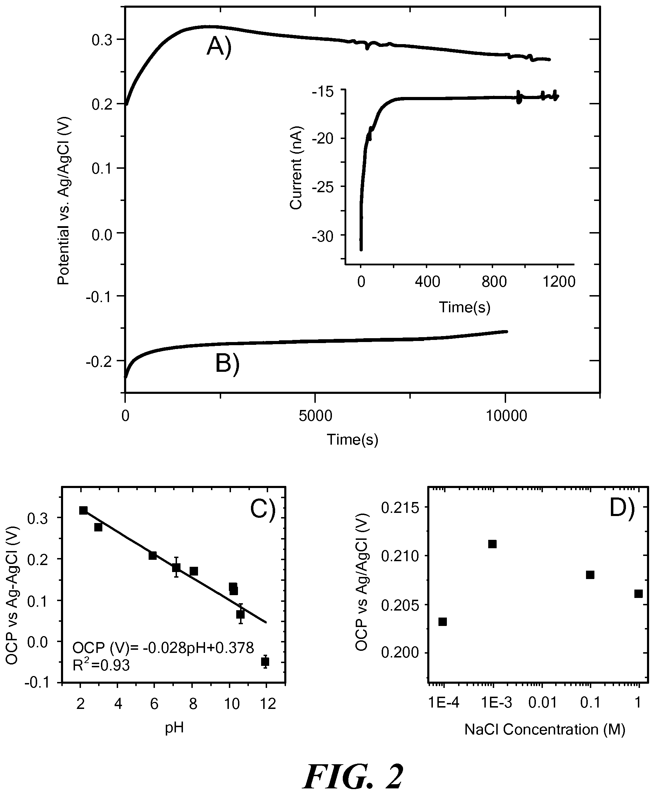

FIGS. 2 A-D are graphs showing open cell potential (OPC) of a palladium hydride (PdH) electrode under various conditions. FIG. 2A shows OPC of a PdH electrode as a function of time. The electrode was not charged in 2 N HCl. FIG. 2B shows OPC of a PdH electrode as a function of time. The electrode was charged in 2 N HCl. Inset shows electrochemical charging of a Pd electrode at -0.375 V for 20 min in 2 N HCl. FIG. 2 C shows OPC of a semi-charged PdH electrode as a function of pH. FIG. 2D shows OPC of a semi-charged PdH as a function of NaCl concentration in 100 mM phosphate buffer (PB; pH 7).

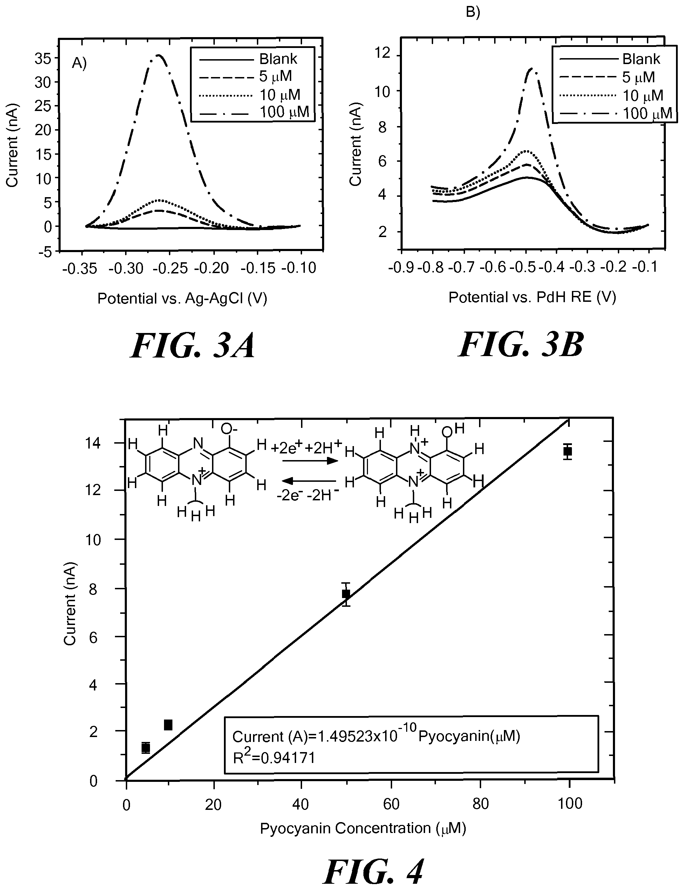

FIGS. 3 A-B are graphs showing square wave voltammograms for measuring currents from defined amounts of pyocyanin using two different reference electrodes. FIG. 3A shows square wave voltammograms of 0 (Blank), 5, 10 and 100 $\mu$M pyocyanin in 100 mM (PB from -0.350 to -0.100 V vs. Ag/AgCl reference electrode. FIG. 3B shows square wave voltammograms of 0 (Blank), 5, 10 and 100 $\mu$M pyocyanin in 100 mM PB from -0.800 to - 0.100 V versus a PdH reference electrode. Scans were performed at an amplitude voltage of 0.025 volts and a frequency of 15 Hz.

FIG. 4 is a graph of variation in current as a function of the concentration of pyocyanin, measured using a device having a nanofluidic electrode assembly as described in FIG. 1. The graph correlates maximum current detected from solutions containing pyocyanin in the concentration range of 0 to 100 $\mu$M in 100 mM PB. The current from the 0 $\mu$M pyocyanin (blank) was subtracted, so that at a solution containing no pyocyanin has a current of 0 A. Inset shows oxidized and reduced forms of pyocyanin.

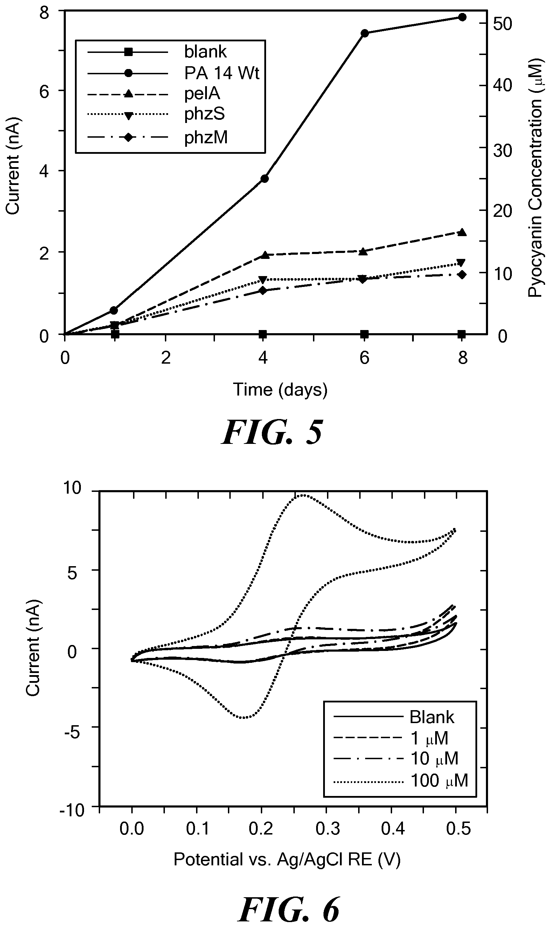

FIG. 5 is a graph of current produced by pyocyanin present in culture medium of the *Pseudomonas aeruginosa* strains, PA14 (wild type), pelA, *phz*S, and *phz*M at specific time points during a period of 8 days of culture at 37 °C. The pelA strain lacks genes necessary for producing robust biofilms, and the *phz*S and *phz*M strains lack genes necessary for pyocyanin production, but are able to synthesize precursors of pyocyanin. The Y-axis on the right shows pyocyanin concentration.

FIG. 6 is a graph of cyclic voltammograms for 0 (Blank), 1, 10, and 100 $\mu$M ferrocene dimethanol in 1 M KCl solution. The scan rate was 0.050 V/s, and the voltage interval was 0 to 0.750 V using a Ag/AgCl reference electrode.

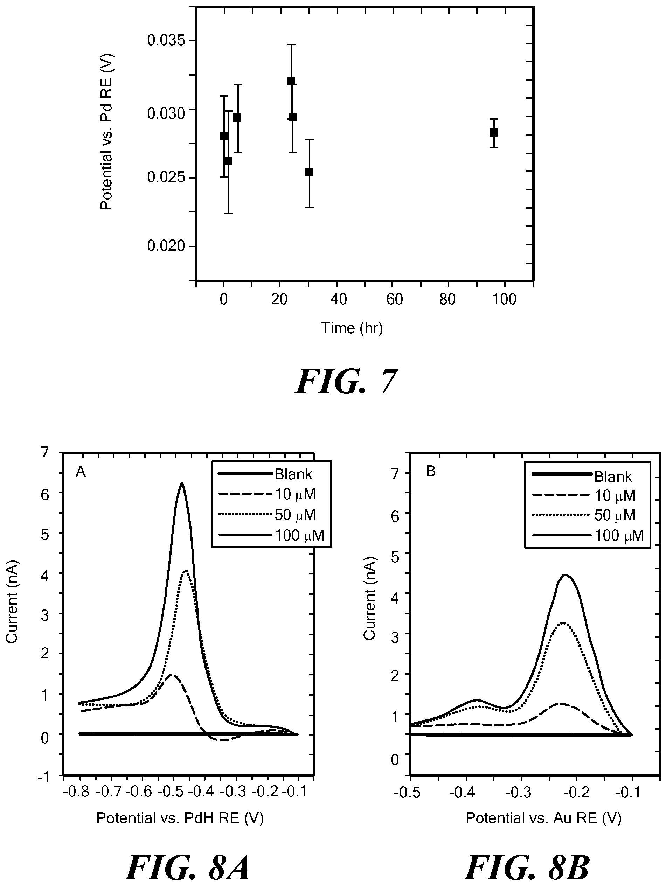

FIG. 7 is a graph of oxidation potential of ferrocene dimethanol versus a semi-charged PdH reference electrode measured as a function of time (hours).

FIGS. 8 A-B are two graphs of differential pulse voltammograms of different concentrations of pyocyanin measured using different reference electrodes. FIG. 8A is a graph showing voltammograms of 0 (Blank), 10, 50, and 100 $\mu$M pyocyanin in 100 mM PB in the voltage interval of -0.800 to -0.100 V versus PdH reference electrode. FIG. 8B is a graph showing voltammograms of 0 (Blank), 10, 50, and 100 $\mu$M pyocyanin in 100 mM PB in the voltage interval of -0.500 to -0.100 V versus Au reference electrode. All scans were performed at an amplitude voltage of 0.05 volts and a frequency of 15 Hz.

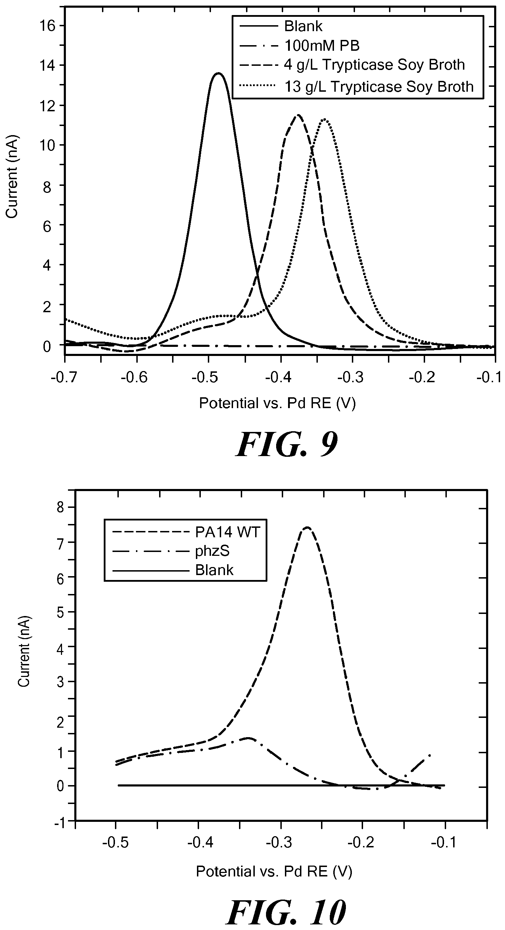

FIG. 9 is a graph of differential pulse voltammograms of 100 $\mu$M pyocyanin in PB (left curve) and in 4 g/L (middle curve) and 13 g/L (right curve) of Trypticase Soy Broth. The scans were performed at an amplitude voltage of 0.05 V and a frequency of 15 Hz. The horizontal line at the bottom is a voltammogram obtained with the culture medium alone.

FIG. 10 is a graph of differential pulse voltammograms of wild type PA14 and *phz*S after 6 days of growth at 37 °C. Scans were performed from -0.5 to -0.1 V at an amplitude voltage of 0.05 V and a frequency of 15 Hz. The horizontal line at the bottom is a voltammogram obtained with the culture medium alone.

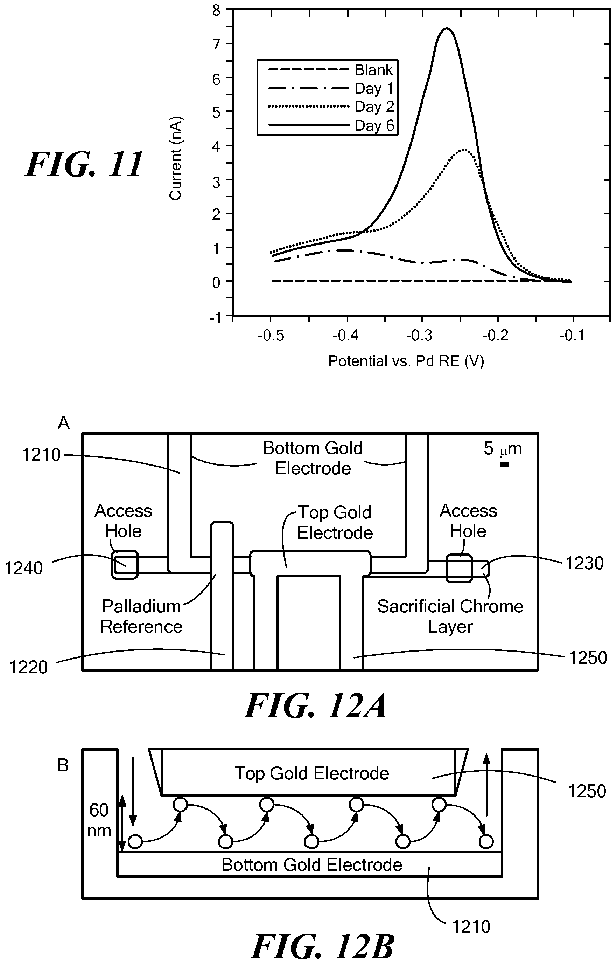

FIG. 11 is a graph of differential pulse voltammograms obtained using supernatants of a culture of PA14 collected at days 1, 4 and 6. Scans were performed from -0.5 to -0.1 V at an amplitude voltage of 0.05 volts and a frequency of 15 Hz. The horizontal line at the bottom is a voltammogram obtained with the culture medium alone.

FIGS. 12A and B are optical micrographs of a microfabricated nanofluidic electrode assembly, and a schematic representation of redox cycling in the nanofluidic electrode assembly. FIG. 12A shows that removal of the sacrificial chrome layer leaves behind a 60 nm gap between the bottom electrode and the Pd reference, and also between Pd reference and the top electrode. FIG. 12B shows a representation of redox cycling in the device of FIG 12A. A reduced molecule (near bottom electrode) enters the nanofluidic channel and becomes oxidized and moves toward the top electrode. The small spacing leads to multiple redox reactions before the molecule diffuses out of the channel.

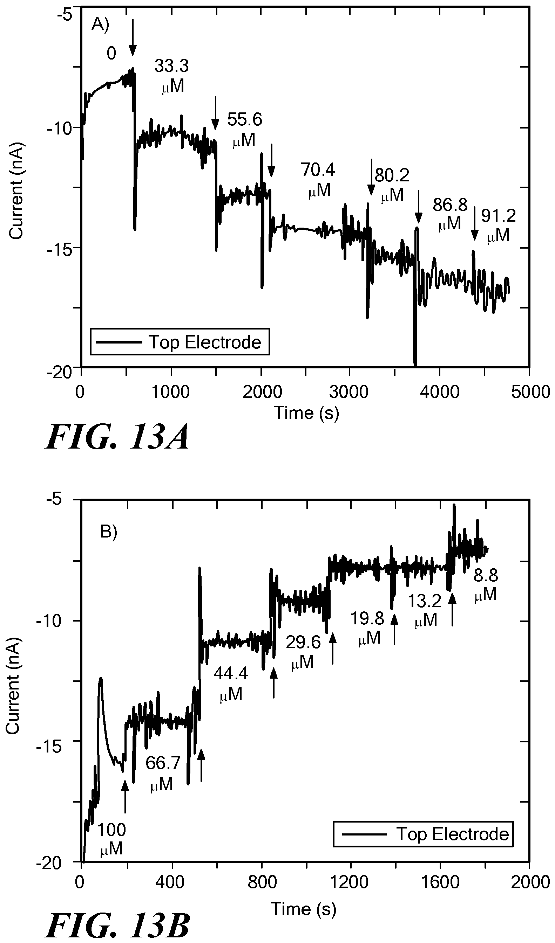

FIGS. 13A and B are graphs of the real-time current (nA) response measured by a nanofluidic electrochemical assembly with an integrated Pd reference electrode and two closely spaced working electrodes as shown in FIG. 12. FIG. 13A and B show the current measured by the top working electrode as pyocyanin concentration is increased or decreased, respectively. Arrows mark the times at which the concentration of pyocyanin in the sample was changed. The top electrode was set to -1.5 V, and the bottom electrode to 0 V versus a Pd reference electrode.

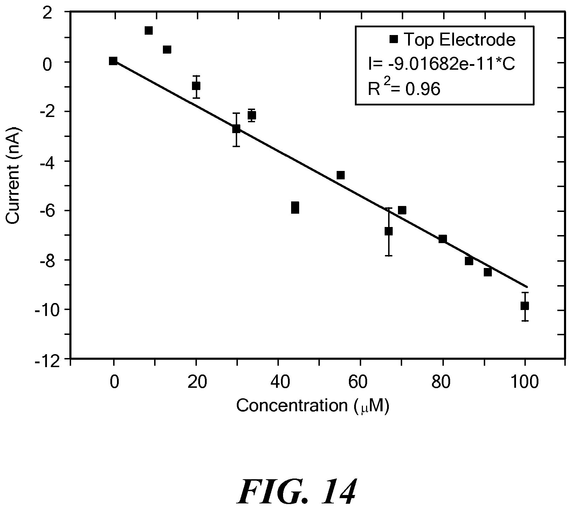

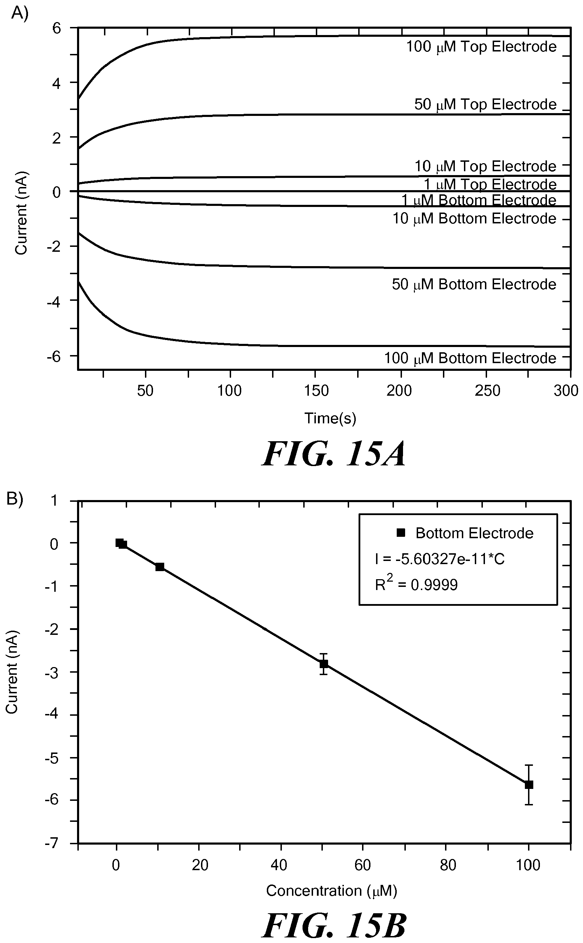

FIG. 14 is a graph of current (nA) measured at the top electrode (FIG. 12) as a function of pyocyanin concentration ($\mu$M). The top electrode was set to -1.5 V, and the bottom electrode to 0 V versus a Pd reference electrode. After steady state current was attained, at each pyocyanin concentration the current response was measured for 25 seconds, and an average was taken. The error bars represent one standard deviation from the average steady state current that was measured for each pyocyanin concentration. FIGS. 15A and B are graphs of real-time current response measured by a nanofluidic electrochemical assembly with an external reference electrode and an electrically isolated setup. FIG. 15A shows current (nA) measured in samples containing 0 $\mu$M (control; fourth curve from the top), 1 $\mu$M (fourth curve from the top), 10 $\mu$M (third and fifth curves from the top), 50 $\mu$M (second and sixth curves from the top), and 100 $\mu$M (the top and the bottom curves) pyocyanin in trypticase soy broth. FIG. 15B shows variation in current measured at the bottom gold electrode as a function of concentration. The bottom electrode was set to -0.35 V reducing potential while the top gold electrode was set to -0 V oxidizing potential versus a Ag/AgCl (1 M KCl) reference electrode. About 25 seconds of current response was averaged at each pyocyanin concentration after steady state current was attained. The error bars represent one standard deviation from the average steady state current that was measured for each pyocyanin concentration during three separate measurements.

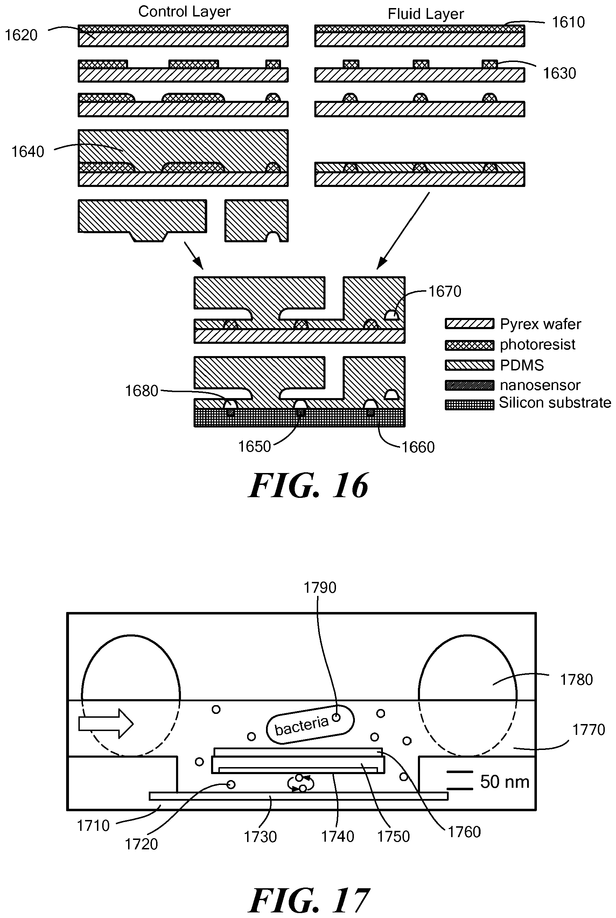

FIG. 16 is a fabrication scheme for a microfluidic platform showing the separate preparation of two different layers, a control layer and a fluid layer, followed by a combination of the two to produce microfluidic channels, and placing of the combination on nanofluidic assembly sensors.

FIG. 17 is a schematic diagram (side view) of a single cell capture and detection chamber (not to scale). The cells are imaged from the top using a stereomicroscope as electrochemical information is being obtained.

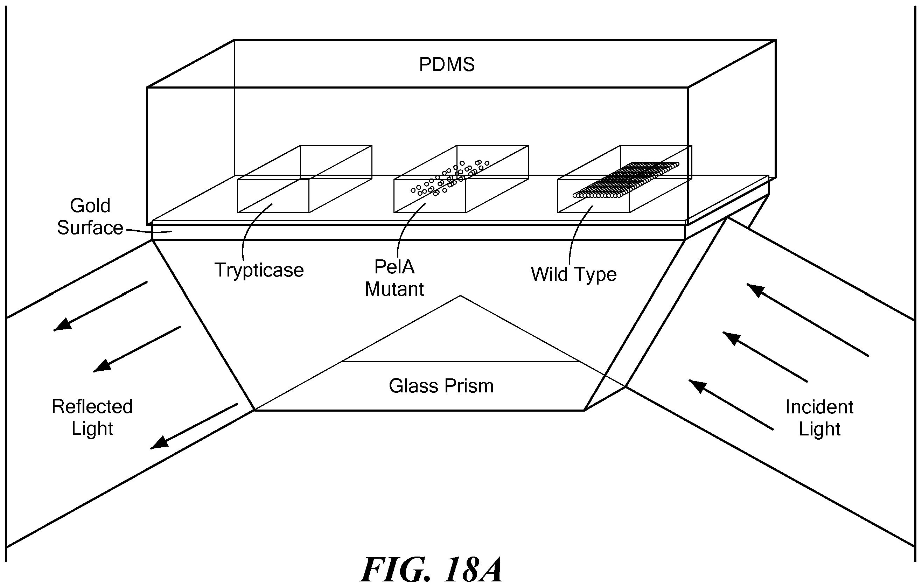

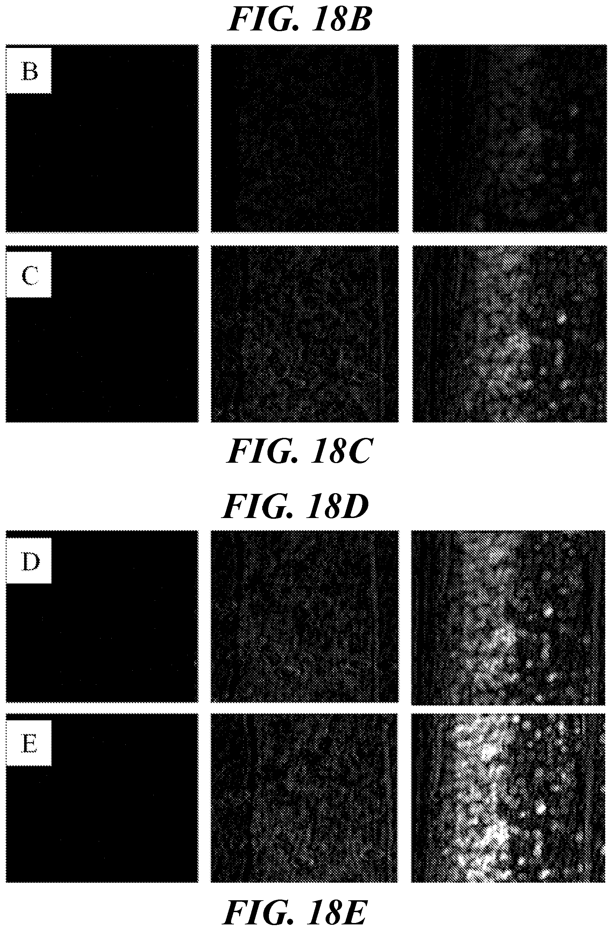

FIGS. 18 A-E show a set up for surface plasmon resonance imaging (SPRI) measurements, and SPRI difference images. FIG. 18A shows PDMS having three microfluidic channels that are reversibly sealed against a high refractive index glass prism coated with 50 nm of gold. The left channel is filled with trypticase, the middle channel is filled with pelA mutant, and the right channel is filled with wild type *P. aeruginosa,* PA14. The setup is placed inside an SPR imaging system. FIGS. 18 B-E show SPRI difference images after (B) 4 min, (C) 8 min, (D) 12 min, and (E) 18 min. Images in each column correspond to the left, middle, and right microfluidic channels, respectively.

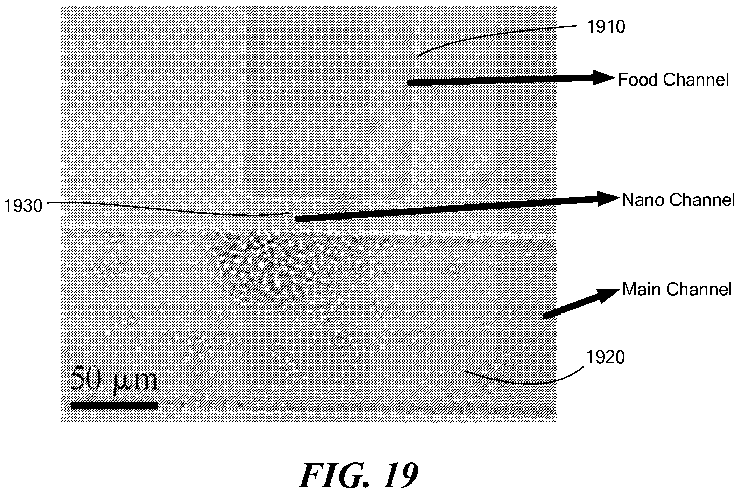

FIG. 19 is an optical micrograph image of bacteria forming a biofilm at the entrance of a nanochannel connecting two microchannels made from PDMS. The bacteria are attracted to the fresh growth medium diffusing through the nanochannel, but cannot squeeze through to get to the food source.

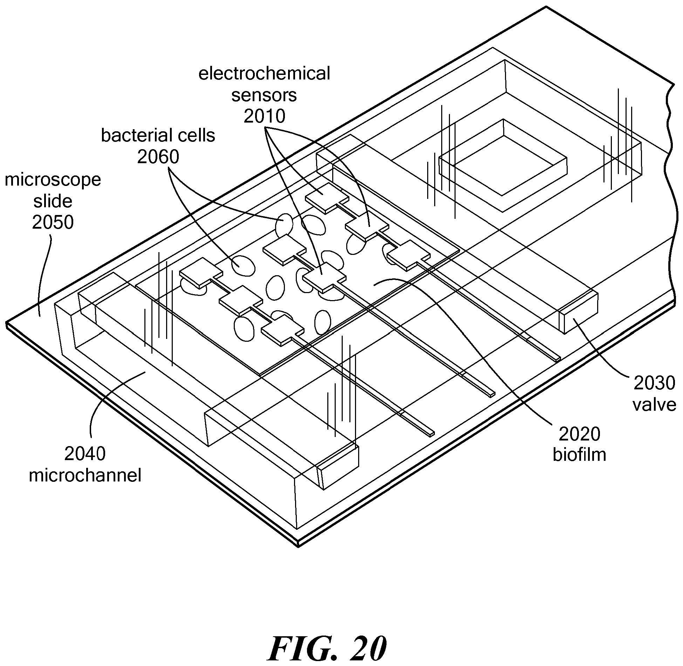

FIG. 20 is a schematic diagram of a nanoscale electrochemical sensor array integrated into a microscope slide that creates the top wall of a microfluidic channel. The sensors selectively detect redox-active molecules as they diffuse through a biofilm from the bulk solution. In the schematic, the top surface is the microscope slide with nano-electrochemical sensors.

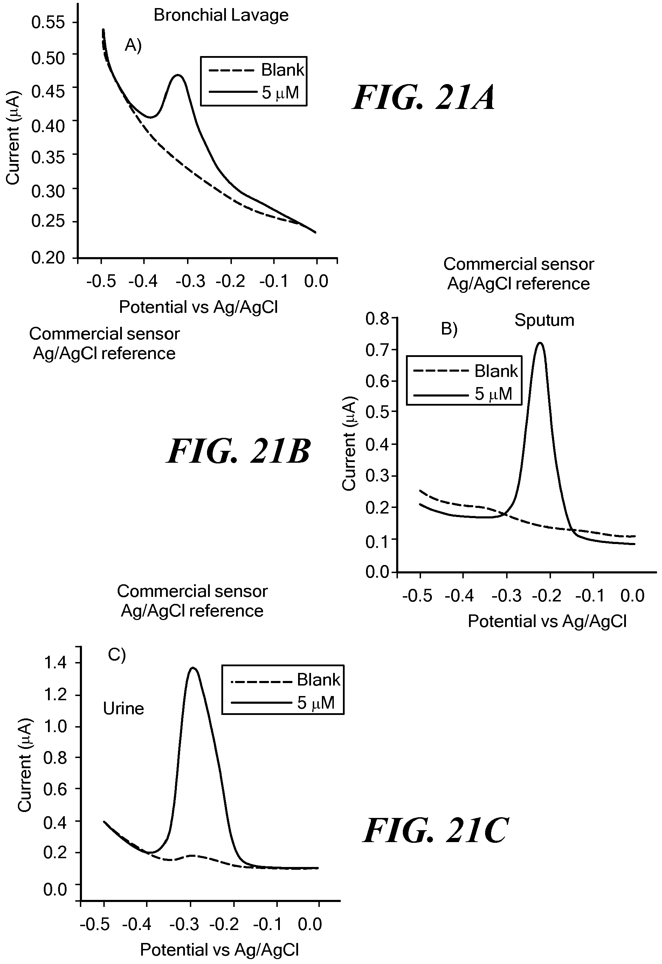

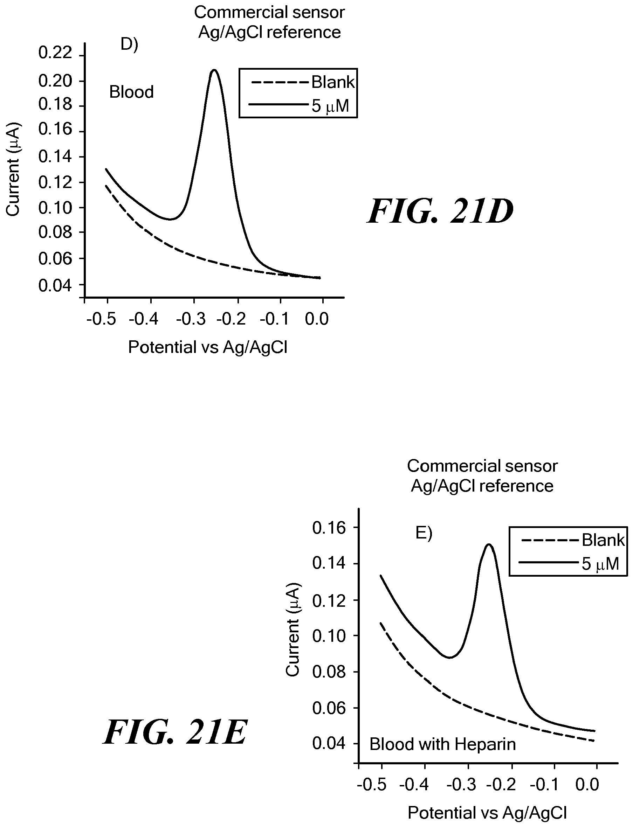

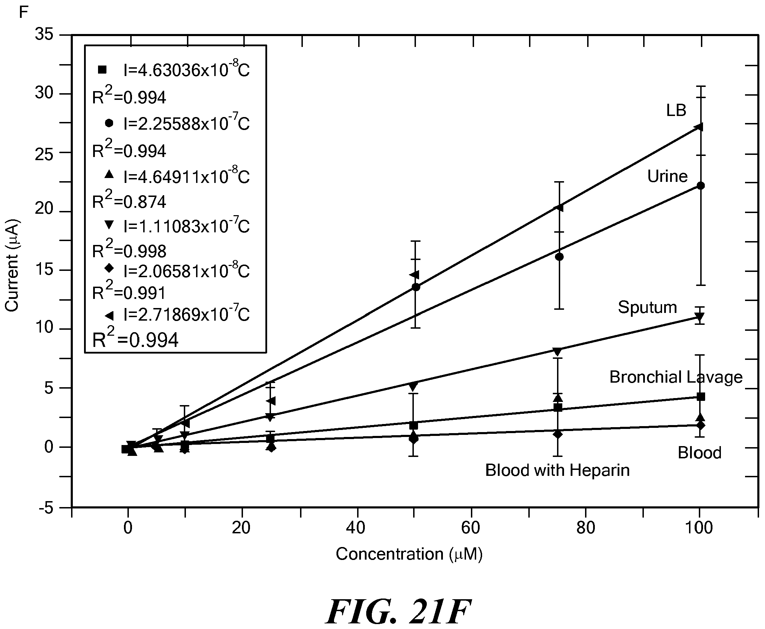

FIGS. 21 A-E are graphs of current measured for detecting pyocyanin in biological fluids using a commercial sensor having an Ag/AgCl reference electrode, a working electrode, and a counter electrode. Defined amounts of pyocyanin were added to the biological fluids. The various biological fluids are bronchial lavage (A), sputum (B), urine (C), blood (D) and blood with heparin (E). FIG. 21F is a graph showing a linear relationship between the current measured and concentration of pyocyanin in different biological fluids.

## DETAILED DESCRIPTION OF THE DISCLOSURE

The present invention is as defined in the claims.

The present disclosure provides devices for highly sensitive electrochemical detection of redox active substances in picoliter volumes of liquid sample. The devices use a nanofluidic electrode assembly having all required electrodes (i.e., reference electrode, one or more working electrodes, and optional counter electrode) integrated within a nanofluidic channel. The disclosure further provides sensor devices that incorporate the nanofluidic electrode assembly, combinations of such sensor devices, medical devices incorporating the sensor devices, as well as methods for fabricating and using the devices.

Methods of the disclosure include methods for the fabrication of a microscale or nanoscale palladium hydride reference electrode and its integration inside of a nanoscale constriction (nanofluidic channel) along with a working electrode, such as a gold working electrode, to create a complete electrochemical sensor device. After charging the palladium electrode with hydrogen, the device can be used to detect redox active agents (e.g., pyocyanin produced by *Pseudomonas aeruginosa*) at concentrations from 1-100 $\mu$M, with a detection limit of less than 1 $\mu$M. This is the first example of a palladium hydride reference electrode integrated with a micro fabricated electrochemical sensor in a nanofluidic setup. In methods of using the device, a redox active agent can bemeasured over the course of several days, e.g., to monitor bacterial growth. By utilizing suitable voltammetry protocols, a redox active moleculecan be selectively detected in a complex solution without the use of any electrode surface modification. In alternative embodiments according to the present disclosure, the working electrode is functionalized in order to increase the selectivity of detection.

Microfabricated nanofluidic electrode assemblies having an integrated palladium reference and two closely spaced working electrodes (an oxidizing working electrode and a reducing working electrode) inside the device nanochannel are also described herein. One such device has been used to monitor changes in pyocyanin concentration amperometrically in real time. A linear response in faradaic current ($R^2$ = 0.96) was observed over a biomedically relevant range of pyocyanin concentrations (0-100 $\mu$M) while continuously measuring the current for 2 hours. Measurement of the current that results from the repeated oxidation and reduction of pyocyanin at two closely spaced electrodes inside the device nanochannel yielded a 1.07 $\mu$M limit of detection without electrical isolation of the electrochemical cell. Since a reference electrode is integrated inside the nanofluidic channel of these sensors, they can potentially be employed to detect pyocyanin and other redox-active molecules in wide range of medical and environmental settings where space is limited, and without the risk of a biofilm forming on the reference or other electrodes. Nanofluidic electrode assemblies can also be used with an external Ag/AgCl reference electrode, e.g., to determine the concentration of a redox active agent in a solution, such as a patient sample or a culture medium. This type of analysis is completed in less than two minutes and also has a sub micromolar detection limit.

Amperometric detection provides a simple means of measuring the concentration of an electro-active species in solution. This is done by applying to an electrode a potential that oxidizes or reduces the molecule of interest. As the molecule of interest interacts with the electrode surface, one or more electrons are transferred between the two, resulting in the flow of a measurable current. Numerous compounds, including histamine, glutathione and dopamine, have been successfully detected using this approach (Lacher et al., 2001, Electrophoresis 22, 2526-2536; Dam et al., 2007, Analyst 132, 365-370; Pihel et al., 1995, Anal Chem 67, 4514-4521). However, often, the molecule of interest is in a complex medium that contains multiple electro-active species that react at the same potential, thus requiring that electrochemical detection be coupled with a separation technique, such as liquid chromatography (LC) or capillary electrophoresis (CE). While improving selectivity and sensitivity, these approaches also require additional processing time and reagents, and increase the device footprint. In certain situations, this can be avoided. If the molecule of interest is stable in both its oxidized and reduced form, a technique known as redox cycling can be employed to amplify the measured current over unstable molecules.

Redox cycling typically uses two working electrodes, with the potential of one electrode set to oxidize the target molecule, while the second electrode is set to a reducing potential. When the target molecule interacts with the oxidizing electrode it donates an electron, and when it interacts with the reducing electrode it gains the electron back and is able to repeat the process (FIG. 12). This cycling process significantly increases the signal compared to a single working electrode system. Straver *et al.* (2012) recently demonstrated this effect by constructing a micro fluidic electrode assembly where the spacing between the top and bottom electrodes was on the order of 1 micron (Straver, et al., Lab Chip 12, 1548-1553).

The theoretical limiting current for a given concentration of an electrochemical molecule undergoing redox cycling in a confined channel is determined by

$$i = \frac{nFSDC}{z} \tag{1}$$

Where $i$ is the limiting current, $n$ is the number of electrons being transferred, F is Faraday's constant, $S$ is the surface area that overlaps between the two electrodes, $D$ is the diffusion constant of the molecule being studied, $C$ is the concentration of the redox species, and $z$ is the distance between the two electrodes (Goluch et al. 2009, Anal Bioanal Chem 394, 447-456). Equation 1 shows that for a given molecule, the net current measured at an electrode can be increased by either increasing the area that overlaps between electrodes or decreasing the spacing between electrodes. Decreasing the vertical spacing between the two electrodes (i.e. the two electrodes are placed on top of one another), increases the net current response of these devices. The benefit of decreasing the spacing between electrodes over increasing the area of overlap is that it allows for smaller device footprints.

Electrochemical detection systems utilizing redox cycling in nanofluidic channels have been shown to have increased sensitivity over their large scale counterparts, and have even been shown to detect single molecules (Zevenbergen et al., 2007, Nano Lett 7, 384-388); Zevenbergen et al., 2011, Nano Lett 11, 2881-2886). The ease of use of this method lends itself to the detection of biologically relevant molecules (Goluch et al., 2009, Anal Bioanal Chem 394, 447-456; Wolfrum, et al., 2008 Anal Chem 80, 972-977; Webster and Goluch, 2012 Lab Chip 12, 5195-5201). However, none of these earlier studies has utilized an integrated reference electrode. In contrast, the present disclosure provides a microfabricated electrochemical sensor with an integrated reference electrode. The entire footprint of the sensor, including the entire electrode assembly, can be, for example, as small as approximately 15 $\mu$m by 250 $\mu$m.

An electrochemical cell used for potentiometric analysis typically consists of three electrodes: a working electrode, a reference electrode, and a counter electrode, which are used to measure the current at a given potential. Measurements are made at the surface of the working electrode and the resulting potential is compared against the half-reaction taking place at the reference electrode. An ideal reference electrode is constructed from a material that has a high current exchange density, is nonpolarizable, and is not destroyed by the reaction taking place. The counter electrode is employed to ensure that the current passing through the reference electrode is never large enough to damage the electrode. When small currents are measured at the working electrode, such as less than 1 nA, the reference electrode can also serve as a counter electrode, allowing a two electrode setup. The current flowing through the working electrode, which can be measured using an amperometry circuit that is part of the device or a separate component, can be used as a measure of the concentration or amount of the selected analyte.

By decreasing the size of the electrode assembly, many individually addressable electrode assemblies can be packaged onto a single detection platform. By constructing electrode assemblies spaced less than 100 nm apart, it has been shown that the sensitivity of electrochemical devices can be increased several-fold as a result of the short molecular transport times between electrodes in these confined spaces (Wolfrum et al., 2008, Anal Chem, 80, 972-977; Zevenbergen et al., 2007, Nano Lett, 7, 384-388; Zevenbergen et al., 2011, Nano Lett, 11, 2881-2886). However, miniaturization, stability, and reliability of the reference electrode has not been addressed prior to the present disclosure.

Macroscale reference electrodes can be used in tandem with micro and nanofluidic electrodes. However, IR drop, which is based on the distance between the reference electrode and working electrode, can lead to errors in the measured currents, which negatively impacts device sensitivity. Scale down is further impeded by the dissolution of the electrolyte solution that many reference electrodes employ to increase their stability. For instance, a silver/silver chloride (Ag/AgCl) reference electrode consists of a silver wire that is coated with silver chloride. The wire is usually surrounded by a solution saturated with chloride ions to maintain a constant potential at the reference electrode. When placed into a test solution small amounts of the chemicals can diffuse through the barrier, but since the solution is saturated, the Ag/AgCl reference electrode is not affected by this small change. However when a Ag/AgCl electrode is scaled down to the microscale, then the diffusion of even a small amount of the test solution into the reference electrode solution is more pronounced and quickly causes degradation of the reference electrode. Thus, by employing the nanoscale reference electrode of the present disclosure, which fits within a nanofluidic channel, a considerably more stable reference electrode is achieved, providing improved accuracy and durability of the sensor device.

To avoid dissolution issues involved with microscale AgCl surfaces, a different reference material is required. Palladium (Pd), a noble transition metal, is capable of holding up to 900 times its volume of dissolved hydrogen within its crystal structure. It functions in a manner similar to the standard hydrogen reference electrode in that the potential is set by the hydrogen ion concentration versus the amount of absorbed hydrogen molecules this type of reference electrode is also referred to as a palladium hydride (PdH) reference electrode. The potential remains constant as the amount of absorbed hydrogen changes over a wide range of values. By storing the hydrogen within the metal, the electrode stability greatly increases and is only affected very slightly by changes in the solution pH, making it an attractive option for biosensors. The increased stability makes PdH a useful reference electrode material and researchers have focused on optimizing PdH REs for bulk electrochemical measurements (Czerwinski et al., 1991 J Electroanal Chem Interfacial Electrochem, 316, 211-221; Czerwinski and Marassi, 1992, J Electroanaly Chem Interfacial Electrochem, 322, 373-381; Czerwinski et al., 1995, J Electroanal Chem Interfacial Electrochem, 386, 207-211), but not for microfluidic or nanofluidic environments, prior to the present disclosure. Microfabricated Pd structures are often used in sensors to detect hydrogen gas at very low concentrations where the electrode potential is affected by the hydrogen concentration (Zeng et al., 2011, Nano Lett, 11, 262-268). Odijk et al (2009) integrated a microfabricated Pd reference electrode into a micro fluidic device that is employed in oxidizing metabolites in aqueous solution (Odijk et al., 2009, Lab Chip, 9, 1687-1693). However, in the micro fluidic device the electrodes are accessible to large particles such as a bacterial cell which can interact with and attach to the electrodes. In the devices of the present disclosure, however, access of bacterial cells and larger cells to the reference electrode is prevented by placing the reference electrode surface within a nanofluidic channel too small to permit the entry of bacteria or other cells.

Described herein are methods of fabricating miniaturized Pd metal electrodes in a single metal patterning step such that the electrode is integrated inside a nanofluidic sensor assembly. The fabrication can be carried out using standard photolithography and physical deposition techniques. The half-reaction in a PdH reference electrode evades the common problem of electrolyte dissolution in buffered solutions. Miniaturizing the reference electrode allows it to be placed near the working electrode, thus avoiding IR drop. For these reasons a PdH reference electrode was microfabricated inside a nanofluidic cavity along with a working electrode (e.g., a gold working electrode) to obtain a nanofluidic electrode assembly (FIG. 1 A). The nanofluidic electrode assembly was used for testing concentrations of the biologically relevant molecule pyocyanin. An optical micrograph of a completed nanofluidic electrode assembly is shown in FIG. 1A. The nanofluidic electrode assembly includes a working electrode **(120),** a Pd reference electrode **(140),** and access holes **(150).** An advantage of the overall device geometry is that the working electrode is isolated from the bulk fluid, eliminating the need for flow velocity correction that is necessary when integrated within microfluidic channels. A second important benefit is that the entire assembly along with the reference can be integrated inside enclosed fluidic chambers containing only a few picoliters of liquid (roughly the size of a single mammalian cell).

An exemplary use of the nanofluidic electrode assembly described herein is detection of a microbe, such as a human pathogen, by detection of a redox active compound released from the microbe. Ventilator associated pneumonia is a prevalent affliction that plagues many immune compromised patients in hospitals, and high mortality rates have been observed. A significant contributor to ventilator associated pneumonia as well as wound and burn victim infections is the opportunistic pathogen *Pseudomonas aeruginosa* (PA). One of the many toxins produced and secreted by *Pseudomonas aeruginosa* is pyocyanin. It has the ability to oxidize and reduce other molecules and has been shown to induce neutrophil apoptosis (programmed cell death), decreased glutathione levels in epithelial cells, cause oxidative stress to cells, and inhibit the beating of lung cilia, all of which allow bacterial infections to thrive, and increases the prevelance of chronic inefctions.

Currently, the most common method for identification of microbial pathogens is culturing colonies, which takes 24 hours or more. Polymerase chain reaction (PCR) identification is faster, but it still takes over an hour and requires expensive reagents. In the case of PA pulmonary infections, the sputum of patients can contain up to 130 $\mu$M concentrations of pyocyanin, and concentrations in the millimolar range have been reported in ear secretions obtained from patients with PA ear infections. These concentrations make it possible to use pyocyanin as a diagnostic marker in electrochemical sensing techniques.Since the pyocyanin molecule is redox active, it can be detected electrochemically. Carbon electrodes were used by Sharp et al (2010) to selectively detect pyocyanin in bulk PA cultures; however the large size of the electrodes does not lend itself to microfluidic systems (Sharp et al., 2010, Bioelectrochem, 77, 114-119). On the other hand, embodiments according to the present disclosure of the microfabricated nanoelectrode assembly described herein can be used for monitoring the production of pyocyanin by several strains of PA using only a few hundred microliters of sample. Sensor devices employing such nanoelectrode assemblies can be incorporated into medical devices such as respirators, or disposable components thereof, such as tubes or masks. The sensors can be connected to control or monitoring electronic devices either by direct electrical connections or by wireless transmitter, and can provide important patient monitoring information in real time, or at selected times or time intervals.

Another embodiment according to the present disclosure is a device having a nanofluidic electrochemical assembly with two working electrodes and an integrated palladium reference electrodes. Such a device makes use of redox cycling to provide greater sensitivity. The exemplary redox active substance pyocyanin produced by PA was detected in these devices using redox cycling, which allows continuous real time monitoring of samples containing the bacteria. The devices have been used to measure changes in pyocyanin concentration in a buffered solution in a microwell that is open to the environment and not electrically insulated. Unlike many biological compounds that react at oxidizing potentials, pyocyanin reacts at a fairly negative reducing potential. By judiciously selecting the potentials that are applied to the two working electrodes, it is possible to avoid the effects of other phenazines and redox-active molecules found in bodily fluids (Imokawa, et al., 2006, Anal Chem, 78, 265-271; Zeng et al., 2011, Nano Lett, 11, 262-268). Using the device of this embodiment according to the present disclosure, pyocyanin concentration was measured also in trypticase soy broth. For these measurements an external Ag/AgCl electrode was used in conjunction with the two working electrode nanofluidic electrode assembly device. The device setup was electrically isolated from the environment, and the potentials turned off between samples. In contrast to methods involving cell culture and genetic identification, concentration measurements for detection of *P. aeruginosa* can be carried out using the nanoelectrode assembly device described herein in less than 2 minutes.

EXAMPLES

Example 1: Solution preparation

Solutions were prepared with ultra-purified water from a Milli-Q system (Millipore resistance > 18 Mohms). Phosphate buffer (PB), for use as supporting electrolyte, was prepared as a 1 M stock concentration from sodium phosphate dibasic heptahydrate and sodium phosphate monobasic anhydrous (Fisher Cat. BP331-1 and Cat. BP329-1, respectively) at pH 6.8. Pyocyanin was purchased as a solid from Cayman Chemicals (Cat. 10009594) and dissolved in Milli-Q water to make 150 $\mu$M stock solutions. The stock pyocyanin solutions were then diluted with phosphate buffer to make samples for testing. As a confirmation that the constructed devices were functioning properly, ferrocene dimethanol (Sigma-Aldrich Cat. 372625) was dissolved in 1 M KCl (Fisher Cat. P330-500) at concentrations ranging from 1 to 1000 $\mu$M (see, e.g., FIG. 6). Hydrochloric acid (HCl; 37 %; Fisher Cat. AC42379-5000) was used to make a 2 N HCl solution in purified water for PdH reference electrode charging and testing.

Example 2: Fabrication of nanoelectrode assembly sensor with integrated reference electrode

A scheme for the fabrication of a nanoelectrode assembly sensor with integrated reference electrode is shown in FIG. 1B. Three-inch single-side-polished silicon wafers were purchased from University Wafers (UW3P100). Onto these wafers 500 nm of silicon dioxide (**110**) was thermally grown. Devices were constructed using standard optical photolithography and metal deposition techniques. Negative photoresist AZ 2020 (Captial Scietific Cat. AZNLOF2020-1G) was used for all photolithography steps. Metal patterning was achieved using metal lift-off, where the photoresist was first patterned and developed followed by metal deposition and removal of the remaining photoresist in a hot acetone bath.

Metal deposition was accomplished using an electron beam physical vapor deposition system that operated at 9 kV and currents of 15, 45, 50, and 120 mA for chromium (Cr), titanium (Ti), palladium (Pd), and gold (Au) deposition, respectively. The bottom electrode was constructed by depositing 5 nm of Ti, 20 nm of Au (**120**), and 2 nm of Cr. The sacrificial Cr layer (**130**) was constructed by depositing 56 nm of Cr on top of the bottom electrode (**120**). The Pd electrode (**140**) was aligned over the sacrificial Cr layer and bottom electrode, and was fabricated by depositing 2 nm of Cr, followed by 120 nm of Pd and 5 nm of Ti. 500 nm of silicon dioxide (**110**) was plasma sputtered to insulate the devices. Access holes (**150**) were etched down to the sacrificial Cr layer using inductively coupled plasma (Unaxis ICP Etch PlasmaTherm 790) at an RF1 and RF2 power of 250 and 300 watts respectively with an argon and sulfur hexafluoride flow rate of 4.0 and 6.0 sccm, respectively, for 3 minutes. In FIG. 1A, the Au electrode is shown in the outer edge, the Pd wire is in the center, and the access holes shown between the gold and the Au electrode and the Pd are 25 $\mu$m$^2$ squares in cross section.

Electrical connections were made by wire bonding the finished micro fabricated devices to a USB connector (West Bond). Removal of the sacrificial Cr layer was accomplished using chromium etchant (Transene 1020AC) at room temperature. Upon removal of the sacrificial layer an open space (**160**) was created between the bottom electrode and the Pd electrode, forming the lumen of the nanofluidic channel. Removal of the Cr layer was monitored by using the Au electrode as the working electrode and the Pd electrode as the reference/counter electrode and measuring current as a function of time at a potential of 0.005 V. As the chromium metal is removed the resistance between the circuit increases, leading to a decrease in current. The remaining chromium etchant was washed out with deionized water before testing the device further. This fabrication process created the entire nanoelectrode assembly. FIG. 1C shows schematically the entry of redox active substance (**170**) into the nanoelectrode assembly.

Example 3: Electrochemical Measurements

Devices fabricated according to Example 2 were reversibly bonded to the bottom of a polydimethysiloxane (PDMS) well, which contained a 500 $\mu$m diameter hole in the bottom, forming a water-tight seal. The sample volume of the wells was approximately 200 $\mu$L. An Ag/AgCl reference electrode was placed in the solution through the top of the well during the characterization of the PdH reference electrode.

Electrochemical measurements were performed using the devices. To control the potentials applied to the nanoelectrode assembly a bipotentiostat (CHI842C) was utilized. For palladium reference electrode characterization a Ag/AgCl electrode (1 M KCl) (CHI111P) was used. To complete the electrochemical cell a platinum (Pt) counter electrode (CHI115) was employed. To ensure that the devices were functioning as expected, cyclic voltammograms of 1-100 $\mu$M ferrocene dimethanol versus Ag/AgCl reference electrode were obtained (see Example 4 and FIG. 6). The devices were cleaned and stored in deionized water in between electrochemical measurements. Measurement of open cell potential versus pH was done using a freshly etched device in 100 mM phosphate buffer that had its pH adjusted using $H_2SO_4$ and NaOH. The values were recorded once the measured potential reached a stable plateau after approximately 5 min. Salt dependence was measured using NaCl concentrations ranging from 100 $\mu$M to 1 M dissolved in 100 mM phosphate buffer (pH 7). The results are shown in FIG. 2.

Square wave and differential pulse voltammetry, which are more sensitive measurement techniques than cyclic voltammetry, were used for pyocyanin measurements. A counter electrode was not included in the micro fabricated nanoelectrode assembly. However, the construction of microfabricated Au and Pt electrodes has been well documented and could be added with one extra photolithography step Goluch et al., 2009, Anal Bioanal Chem, 2009, 394, 447-456; Silva et al., 2009, J Brazil Chem Soc, 20, 1235-1241; Chung-Chiun, 1993, Appl Biochem Biotech, 41, 99-107.

Example 4: Testing of completed nanoelectrode assembly devices using ferrocene dimethanol

Testing of the completed devices was performed using ferrocene dimethanol in concentrations ranging from 1-100 $\mu$M dissolved in 1 M KCl solution (FIG. 6). The test solutions were scanned from 0 to 0.5 V versus a Ag/AgCl (1 M KCl) reference electrode using cyclic voltammetry. The PDMS well was washed three times with blank KCl solution between each test to ensure absence of cross contamination. For these tests, only the gold working electrode was utilized as a means of establishing that the device was functioning. Ferrocene dimethanol was chosen because its electrochemistry has been widely studied. The half wave potential, or the point where the current is half of the limiting current is shown in FIG. 6 to be about 250 mV which matches values reported in literature.

Example 5: Testing the stability of palladium reference electrode

To determine the stability of the constructed reference electrode with time, differential pulse voltammograms of ferrocene dimethanol were measured at different time intervals. Over a 4 day period of measurements the oxidation potential of ferrocene dimethanol versus the PdH reference electrode was found to be approximately 28 mV with a standard error of only 2.2 mV. (FIG. 7). The expected oxidation potential was approximately 220 mV versus a Ag/AgCl reference electrode. The measured potential difference was approximately 190 mV, which agreed with the measured potential difference seen in pyocyanin measurements.

Example 6: Testing of Microfabricated devices and PdH referenc electrode

Once the chromium in the sacrificial layer was removed, the PdH electrode was used as a working electrode and charged at -0.375 V vs. a Ag/AgCl reference electrode in 2 N HCl for 20 minutes. This was done to load the Pd electrode with hydrogen to stabilize its potential allowing it to be used as a reference electrode. After charging, the open circuit potential (OCP) versus time was monitored to determine the stability of the PdH reference electrode in 2 N HCl. As can be seen in FIG. 2, after charging, the OCP increases initially and then remains at a constant value of approximately -0.160 V versus a Ag/AgCl reference electrode for almost 1.0 hour. The potential for this fully charged palladium hydride reference electrodes is approximately 0.050 V versus a standard hydrogen electrode. Taking into consideration the potential contributed by the Ag/AgCl reference electrode, the measured potential (FIG. 2 B) of the PdH electrode is consistent with literature values. Furthermore, the measured potential closely matches also the value of the potential reported by Chassaniol et al (2011) for their commercially available charged palladium reference electrodes at a similar pH (Chassaniol et al., Thermo Scientific, Instrument application document. 15). In FIG. 2 B the potential was constant for about an hour after it reached -0.160 V. Therefore, there was a possibility of the potential drifting after an hour. This level of stability was expected as all experiments were performed in an oxygenated solution of 2 N HCl. Oxygenated solutions are known to cause decreased stability in palladium hydride systems. For many single use devices, such as the one described in Example 2, the cyclic, square wave, and differential pulse voltammetry measurements take much less than an hour to complete, indicating that the PdH reference electrode performance was adequate.

The fully charged PdH reference converted to a semi-charged state after a few hours, even without use, resulting in a significant shift of its open cell potential versus Ag/AgCl (FIG. 2A). FIG. 2A shows that a palladium electrode without the initial 20 min charging step at -0.375 V approaches an OPC of +0.300 V vs. Ag/AgCl in 2 N HCl. The semi-charged state is significantly more stable, with measured potentials shifting less than 10 mV over the course of 96 hours for ferrocene dimethanol (FIG. 7). The state is distinct from both uncharged and fully charged reference electrodes.

The pH and NaCl concentration dependence were measured and are shown in FIGs. 2 C and D, respectively. The pH change was measured to be 28 mV/pH unit. The potential at each reported pH was tested three times. Standard deviations of the measurements are shown in FIG. 2C. A departure from the ideal 59 mV/pH unit shift is not unexpected for palladium electrodes. Palladium is known to undergo slow decomposition as hydrogen-palladium phase changes, and the electrode exhibits ideal pH shifts for short periods after extensive cleaning and pre-treatment. The results shown in FIG. 2C, in contrast, represent typical expected pH dependence for a sensor in oxygenated solutions at standard operating conditions. The electrode potential was not affected by changes in salt concentration (FIG. 2 D) since the half-reaction only involves hydrogen absorption.

Example 7: Pyocyanin Testing using an integrated PdH reference electrode

The nanoelectrode assembly was used to detect pyocyanin in phosphate buffer. FIG. 3 compares square wave voltammograms for three concentrations of pyocyanin in 100 mM phosphate buffer using a commercial reference (FIG. 3 A) and a microfabricated PdH reference electrode in (FIG. 3 B). The square wave scans were performed at an amplitude voltage of 0.025 V and a frequency of 15 Hz. The difference observed in maximum current between the Ag/AgCl and PdH reference electrodes is the result of using a different device for the two sets of measurements. Typically, a dilution series calibration was carried out for each device before sensing measurements were begun. Variability in maximum currents between batches of fabricated devices was less than one order of magnitude.

Different potential ranges were used in the two sets of experiments to account for the potential difference between the two reference half-reactions. FIG. 3 shows that the pyocyanin oxidation peak versus Ag/AgCl is approximately -0.260 V and approximately - 0.480 V versus a semi-charged PdH. The expected difference between the two peaks for the given reference electrodes, adjusted for pH 6.8, at room temperature, was 0.190 V. The potential difference of 0.220 V between the references is routinely observed for pyocyanin. The extra 0.030 V difference is unique to pyocyanin. The expected 0.190 V potential difference between the references was observed when ferrocene dimethanol was used instead of pyocyanin (FIG. 7). For long term cell culture measurements it is important that a single peak is observed at -0.480 V versus PdH and that its position not change over time.

Differential pulse voltammetry was used to scan pyocyanin solutions (1-100 $\mu$M) in a voltage interval of -0.7 to -0.1 V at an amplitude voltage of 0.05 V and a frequency of 15 Hz. Three samples were measured at each concentration. Current measured with 100 mM phosphate buffer alone was subtracted from each scan. The average maximum current and standard deviation for three measurements at each concentration are shown FIG. 4. A linear fit of the data was also obtained and is shown in FIG. 4. The PDMS wells were rinsed with phosphate buffer between samples. A limit of detection of 0.597 $\mu$M pyocyanin was obtained for this system, which is within an order of magnitude in sensitivity to standard differential pulse voltammetry measurements using an ultra-micro electrode. The limit of detection was calculated as $3\sigma$/sensitivity, where $\sigma$ is the average of the standard deviations from three separate 100 mM phosphate buffer only (blank) measurements ($2.98 \times 10^{-11}$ A), and the sensitivity is the slope of the fitted line.

Example 8: Comparison of pyocyanin detection using different reference electrodes

Two different nanoelectrode assembly devices were compared (FIG. 8) by measuring pyocyanin concentration. The data in FIG. 8A were obtained using a nanoelectrode assembly with a gold working electrode and a semi-charged PdH reference electrode. The data in FIG. 8B were obtained using a nanoelectrode assembly with both a gold working electrode and an integrated gold reference electrode. The bottom electrode was used as the reference and the top was used as the working electrode. A platinum wire was used as a counter electrode in all cases. FIG. 8 shows that the limit of detection was not affected by the type of reference electrode. A similar limit of detection was obtained with both the PdH and Au reference electrodes.

Example 9: Comparison of oxidation potential of pyocyanin in phosphate buffer versus trypticase soy broth

The oxidation potential of pyocyanin in phosphate buffer was compared to that in trypticase soy broth. A significant shift in the oxidation potential was observed when pyocyanin was dissolved in phosphate buffer versus pyocyanin produced by wild type *P. aeruginosa* in trypticase soy broth measured using a Pd reference electrode. The shift in oxidation potential was observed was believed to be associated with components in the trypticase soy broth. Scans of 100 $\mu$M pyocyanin in phosphate buffer and trypticase soy broth were performed. Systematically changing the composition of the solution revealed a gradual shift in oxidation potential for pyocyanin as shown in FIG. 9. Cell culture experiments were performed with trypticase at a concentration of 30 g/L accounting for the observed peak shift. One or more of the chemicals present in the trypticase soy broth may interact with the palladium electrode causing the shift. Given the data presented in FIG. 2D, it appears that NaCl, one of the main components of tyrpticase soy broth, is not the cause the observed shift.

Example 10: Selective detection of pyocyanin produced by *P. aeruginosa*

In order to selectively detect the production of pyocyanin by *P. aeruginosa,* mutant strains (e.g., *phz*S) that are incapable of producing pyocyanin were used as controls. A peak was observed in the selected scan range for all strains. In the case of *phz*S, the size of the peak was significantly smaller than the pyocyanin peak produced by the wild type strain throughout the duration of the experiments and its maximum was shifted approximately 0.075 V. From FIG. 10 it appears that the redox molecule detected around -0.33 V for the *phz*S strain is not pyocyanin but a derivative or precursor thereof. Furthermore, as more positive potentials were scanned, another peak appeared which is not present in the wild type culture at -0.1 V.

Example 11: Stablity of oxidation potential of pyocyanin

Differential pulse voltammograms of PA14 wild type supernatants were recorded over a six day period. Scans were performed from -0.5 to -0.1 V at an amplitude voltage of 0.05 V and a frequency of 15 Hz. A shift of only 0.020 V was observed in the oxidation peak of pyocyanin produced by wild type bacteria over the course of 6 days. The results are shown in FIG. 11.

Example 12: Detection of pyocyanin in *P. aeruginosa* cell cultures

The microfabricated nanoelectrode assembly devices were used to detect pyocyanin produced from *P. aeruginosa* strains PA14 (wild type), pelA, *phz*S, and *phz*M. The strains were grown at 37 °C for a period of 8 days in trypticase soy broth. A single nanoelectrode assembly was charged prior to the start of these tests. For each measurement 400 $\mu$L of solution was removed from each of the liquid cultures and filtered using a 0.2 $\mu$m sterile nylon filter (Fisher Cat. 09-719C). The filtered samples were scanned from -0.5 to -0.1 V using differential pulsed voltammetry to detect the presence of pyocyanin. It was observed that the pyocyanin oxidation peak shifted to more positive potentials in trypticase soy broth compared to phosphate buffer (FIG. 9). It is hypothesized that a component in the trypticase soy broth interacted with the PdH reference electrode leading to this shift, since a shift in oxidation potential was not observed when pyocyanin was detected in phosphate buffer and trypticase soy broth using a commercially available Ag/AgCl reference electrode.
The currents measured in FIG. 4 were compared against the measured currents from the supernatants of the samples of the PA strains to determine pyocyanin concentration. As a control, trypticase soy broth was also scanned. For each measurement, seven scans were performed and the peak value from the final scan was recorded. Each sample was measured three times and the average values were used to show the results (FIG. 5).
As shown in FIG. 5, as the cells grow the current at the pyocyanin oxidation potential increases. This indicates that pyocyanin was being produced by the bacteria being studied. No other peaks were detected over the range of potentials scanned, indicating that there were no other electrochemically active molecules within the growth media. This is important because anything that contributes to the background noise of the solution could impact the sensitivity of the device.
To confirm that pyocyanin was responsible for the increase in current, four strains of PA were studied, in two of which pyocyanin production was inhibited. Strain pelA is capable of producing pyocyanin but does not contain the genes necessary to produce a robust biofilm. It is believed that a biofilm deficient strain would reach a lower cell density than the wild type, thus leading to a lower concentration of pyocyanin. The data in FIG. 5 confirm this interpretation. During the first few days, pelA produced little pyocyanin compared to the wild type. After eight days, the pyocyanin concentration detected for pelA had increased ($\approx$17$\mu$M), but as expected, was still less than the wild type ($\approx$52 $\mu$M).
In the *phz*S and *phz*M strains the necessary genes required to produce pyocyanin have been removed, and little to no current was expected to be detected from the supernatant of these samples. However, this was not the case.

Most likely the current detected in these strains is from another molecule that is produced by these strains which has a redox potential close to pyocyanin. Several pyocyanin precursor molecules can still be produced by these strains. If the pyocyanin deficient strains produced another phenazine with a very similar redox potential compared to pyocyanin additional analysis would be needed to distinguish between the molecules (Wang and Newman, 2008, Environ Sci Tech, 42, 2380-2386). It was observed that the peak produced by *phz*S was at a different potential than the peak generated by the wild type strain, indicating that an electroactive molecule other than pyocyanin was being produced by *phz*S (FIG. 10).

Stability tests for the PdH reference electrode showed that it was not stable for longer than an hour of continuous measurement. Therefore, after an initial charge, measurements, lasting only a few minutes each were made at discrete times during experiments without recharging the electrode. Scans of the wild type samples on different days showed a shift in the oxidation peak potential of only approximately 0.020 V (FIG. 11). This small shift could have been caused by a number of factors. Over time the pH of the solution could have changed, which is known to affect the redox potential of pyocyanin (Wang and Newman, 2008, Environ Sci Tech, 42, 2380-2386). It is expected that as PA consumed nutrients in the growth medium, a more acidic environment would ensue. However, the pH did not change from day to day, indicating that the shift in the oxidation peak potential was not due to a change in pH. Similar to the changes observed between phosphate buffer and trypticase broth (FIG. 9), a shift in the oxidation potential could be caused also by changes in the chemical composition of the solution resulting from growth of the bacteria and the accompanying consumption of compounds present in the solution and production of waste.

Example 13: Fabrication of device with two working electrodes

Electrodes were micro fabricated on 3-inch silicon wafer substrates, on which 500 nm of silicon dioxide ($SiO_2$) was grown thermally to provide electrical insulation. Metal layers were patterned using standard photolithography techniques and a lift-off process. Gold, chromium, gold, and palladium metal layers were deposited in this order using electron beam deposition. The thicknesses of the gold bottom electrode (FIG. 12A **1210**), gold top electrode (FIG. 12A **1250**), and palladium reference electrode(FIG. 12A **1220**) were 20 nm, 120 nm, and 120 nm, respectively. Sixty nm of chromium was used to separate the top and bottom electrodes. After metal deposition 550 nm of SiO2 was deposited to serve as an insulating layer and to provide structural support for the device. Access holes (FIG. 12 A-B **1240**), which reach the chromium layer, were made in the SiO2 using reactive ion etching. Finally, the nanochannel (**1230**) was created by removing the chromium using chromium etchant (Webster and E. D. Goluch, 2012, Lab Chip 12, 5195-5201). An optical micrograph of a finished device is shown in FIG. 12. A polydimethylsiloxane (PDMS) well, with a 60 μL capacity and a hole in the bottom was aligned and reversibly bonded over the sensors. Solutions were added directly to this well for all electrochemical tests.

Example 14: Electrochemical measurements using a device having two working electrodes

Electrochemical measurements were made with a CHI 1040C multi-potentiostat. A potential of -1.5 V and 0 V was applied to the top and bottom working electrodes respectively, and current was measured every 0.1 second for 5000 seconds. The palladium metal served as both the counter and reference electrodes for these experiments. Since only currents up to a few nanoamps were measured in these experiments, a separate counter electrode was not necessary. The large difference between the potentials at the two working electrodes was chosen to ensure that the pyocyanin, which has a half-wave potential of -0.48 V vs. a semi-charged PdH reference, was both oxidized and reduced inside the nanochannel. Previous studies have shown palladium reference electrodes to be stable, and the large potential difference between the potentials at the two working electrodes, while unnecessary, demonstrated the ability of these devices to monitor pyocyanin production by redox cycling. For solitary analysis of sample concentration, an external Ag/AgCl reference electrode (CHI 111P) was used and 0 V was applied to the top working electrode while -0.35 V was applied to the bottom working electrode in the nanoelectrode assembly.

Example 15: Continuous measurements using a device having two working electrodes

The performance of nanoelectrode assemblies according to Example 14 with integrated palladium reference electrodes was characterized using amperometric detection. Pyocyanin was chosen as it is a stable redox-active molecule of significant biological importance.

A microwell made of PDMS was placed on top of a nanoelectrode assembly, aligned with, and attached to the assembly. The microwell was filled with 60 μL of a 100 mM phosphate buffer test sample. To achieve a specific concentration of pyocyanin in the microwell, 20 μL of the buffer was removed and replaced with 20 μL of a phosphate buffer solution containing the required concentration of pyocyanin. During amperometric detection, the top electrode was held at -1.5 V while the bottom electrode was held at 0V versus the palladium reference electrode.

The detections were performed in the manner above because the sensor must remain filled with a solution containing ions during throughout the period of the detections. The sodium and phosphate ions present in the solution screen the charge at the electrode surface. If the nanoelectrode assembly were allowed to run dry, there would be no ions to screen the charge at the electrodes, resulting in an electric field and attractive force that may collapse the nanochannel.

Initially, the microwell was filled with 60 $\mu$L of 100 mM phosphate buffer and the response to the buffer alone control sample (blank) was monitored as a function of time. To show the response of the fabricated devices to different concentrations of pyocyanin, at different time points 20 $\mu$L of sample solution was removed from the well and replaced by 20 $\mu$L of 100 $\mu$M pyocyanin in 100 mM phosphate buffer, followed by agitation. As seen in FIG. 13 changing the concentration of pyocyanin resulted in a nearly instantaneous response.

FIG. 13 shows the results of each of a set of serial increase in concentration (FIG. 13 A) and dilution (B) of pyocyanin. Arrows mark the time points at which the concentration of pyocyanin in the sample was changed. The current measurements were noisy because the experimental setup was not electrically isolated from the environment during the detections to allow easy access to the sample solution.

A total of six additions were made, resulting in sample concentrations of 33.3, 55.6, 70.4, 80.2, 86.8, and 91.2 $\mu$M. As expected, the current response increased linearly as the concentration of pyocyanin in the microwell increased (FIG. 13 A). The current measured at the electrode became more negative with increasing pyocyanin concentration because pyocyanin accepts electrons from the negatively charged electrode thereby lowering the current measured at the electrode.

To determine the response of the device to decreasing pyocyanin concentration the opposite experiment was performed. Starting with a concentration of 100 $\mu$M pyocyanin in 100 mM PB in the microwell, 20 $\mu$L of solution was removed and replaced with 20 $\mu$L of 100 mM PB followed by agitation. This procedure diluted the pyocyanin concentration in the sample. A total of six dilutions were performed (FIG. 13B), which resulted in pyocyanin concentrations of 100, 66.7, 44.4, 29.6, 19.8, 13.2, and 8.8 $\mu$M.

Using Equation 1 the calculated current for 100 $\mu$M pyocyanin in the constructed device is approximately 36.1 nA. The calculated current is almost 4 times larger than that observed and shown in FIG. 14. The difference between the calculated and observed current may be caused by a number of factors. The decreased current may be caused by fouling of the electrodes by pyocyanin, which is known to polymerize at high oxidizing potentials. Another factor could be misalignment of the top electrode with the bottom electrode, which would lead to a smaller area of overlap between the two working electrodes than expected. In addition, removal of the sacrificial layer can lead to buckling of the top electrode upward, increasing the channel height (Zevenbergen et al., 2007, Nano Lett 7, 384-388). Using the same ratio of surface area to buckling (the buckling amount is proportional to the area of the top electrode) obtained by Zevenbergen et al. (2007), the expected current should be 15.4 nA, which is in better agreement with the measured value for 100 $\mu$M pyocyanin (FIG. 14).

The average current for each concentration of pyocyanin was calculated from 25 seconds of steady-state current obtained after sample injection. The error bars shown in FIG. 14 represent one standard deviation from the average steady state current that was measured for each pyocyanin concentration. The average response of the blank was subtracted from each sample and the resulting current versus concentration was plotted for the top electrode (FIG. 14). The limit of detection (calculated as $3\sigma$ / sensitivity, where the sensitivity is the slope of the line in FIG. 14 and $\sigma$ is the standard deviation of the blank measurement) is 1.07 $\mu$M. This value is similar to detection limits reported in literature with other sensors that utilize redox cycling (Kätelhön et al., 2010, Anal Chem 82, 8502-8509; Odijk et al., 2008, Electroanal 20, 463-468). Since the experiments here were run over the course of two hours, some drift in baseline current is expected. Also, a baseline current could not be measured at the end of these experiments as the pyocyanin could not be removed completely from the microwell while a potential difference was applied across the electrodes. This is a possible explanation for why the average current measured at 8.8 and 13.2 $\mu$M pyocyanin was below the current response for the blank sample obtained at the beginning of the experiment.

Example 16: Discrete measurements using a device having two working electrodes

Differences between continuous environmental and single sample laboratory measurements using the nanoelectrode assembly device having two working electrodes was analyzed. Response of the nanoelectrode assembly device sensor to five samples containing 0, 1, 10, 50, and 100 $\mu$M pyocyanin in trypticase soy broth is shown in FIG. 15. Trypticase soy broth is a complex growth media used for bacterial cultures. For these measurements, the intergrated Pd reference was not used. Instead an external Ag/AgCl reference was employed and the setup was electrically isolated from the environment during measurements. The electronics were turned off between measurements and the fluid well was rinsed between samples.

Under controlled experimental conditions, the devices performed very well. A single device was used to obtain the data in FIG. 15, where each of the five samples was measured three times. FIG. 15A shows the currents measured at the two working electrodes for each of the concentrations. The baseline current of the blank control was subtracted from each of the measurements. A steady state current measurement is obtained within 2 minutes of applying potentials to the electrodes. The long transient response is routinely observed in nanofluidic electrochemical devices, and is most likely caused by reversible potential dependent adsorption of molecules on the electrodes.

The average current for each concentration was calculated from 25 seconds of steady-state current that is obtained two minutes after the start of the measurement. The average response of the blank control was subtracted from each sample and the resulting current versus concentration was plotted for the bottom electrode in FIG. 15B. The error bars shown in FIG. 15B represent one standard deviation from the average steady state current that was measured three times for each pyocyanin concentration . The limit of detection (calculated as $3\sigma$ / sensitivity, where the sensitivity is the slope of the line in FIG. 15B, and $\sigma$ is the standard deviation of the blank measurement) is 441 nM. The unique advantage of redox cycling over other electrochemical techniques is that the sensitivity can potentially be improved further by modifying the device geometry (Goluch et al., 2009, Anal Bioanal Chem 394, 447-456).

Example 17: An integrated sensor platform for analysis of biomass

An integrated micro/nanofluidic device that includes electrochemical and plasmonic sensors integrated inside the channels is constructed. These sensors permit *in situ* and *in vitro* analysis of biomass, such as formation of a biofilm by bacteria. Micro/nanofluidic structures are integrated with a 2-dimensional array of electrochemical sensors based on the design shown in FIG. 1D, and a commercial SPRI (surface plasmon resonance imaging) or SERS (surface enhanced raman spectroscopy) systems. An electrochemical sensor is completely encased inside a nanofluidic chamber allowing diffusion of small molecules to the sensing surface while completely preventing contact of the electrodes in the nanofluidic chamber with the cells, and significantly decreasing entry of protein into the nanofluidic chamber. The devices are single use, so cleaning procedures are not necessary. The devices are used to measure biofilms, and to characterize the spatio-temporal changes that biofilms undergo during bacterial growth.

Measurement of biofilm growth in microchannels using surface plasmon resonance imaging is shown in FIG. 18 (Abadian et al., Real-time detection of bacterial biofilm growth using surface plasmon resonance imaging. microTAS 2012, pp. 413-415). SPRI allows measurement of accumulation of biomass on surfaces with high sensitively and high spatial resolution. SPRI provides label-free qualitative and quantitative information about events occurring near a surface (-200 nm) over a relatively large area ($\sim$1 cm$^2$). Microfluidic channels fabricated herein confine particles near the sensor surface, making it possible to observe bacterial activity inside the channels. Cell movement and growth in the microfluidic channels causes local changes in refractive index that are monitored continuously (Yanase etal., 2010, Biosensors and Bioelectronics, 26(2):674-81). FIG 18A shows an exemplary setup for continuously observing biofilm formation inside microfluidic channels.

Polymer nanochannels are constructed that allow efficient transfer of fluid to and from the sensor area while preventing the escape of cells into the nanochannels. Nanochannels are patterned using electron-beam lithography. Figure 19 shows the formation of a biofilm at the entrance of a nanochannel that supplies fresh growth media to the cells. By constructing micro/nanochannels using polydimethylsiloxane (PDMS) polymer, valves are integrated in the microchannels to selectively trap microbes and regulate bulk fluid flow.

Bacterial cells are exposed to different chemical environments and gradients and the phenotypic response is monitored. Initially, *P. aeruginosa* wild-type and mutant strains with biofilm production suppressed, such as pelA, are used. Cultures of each strain are grown and diluted so that each microchamber has only one or a few cells initially. The effects of growth phase on the measurements recorded is taken into account. The number of cells that are present in the microchamber at the beginning of each experiment is recorded. Control experiments are performed to determine if there is any observable difference in the development of biofilms with small variations in starting concentrations ranging from 1 to about 20 cells per chamber. The cells are transported through the microfluidic channels using pressure driven flow. After cells are trapped and fluid exchange takes place between the microfluidic and nanofluidic channels due to diffusion, a redox active chemical of interest produced by the cells reaches a nanochannel, where it is detected electrochemically due to their redox active nature and interaction with a nanofluidic electrode assembly located within the nanochannel.

In the beginning of the measurements the bacterium is contained in minimal medium. Food is then introduced through nanochannels that connect to the microfluidic channel for the delivery of chemical substances to the microchannel. Penicillin and kanamycin, antibiotics that do not kill *P. aeruginosa,* are introduced to check if the antibioteics have any effect on biofilm formation. Next, ampicillin, which is known to kill *P. aeruginosa,* is used. For each antibiotic, the concentration is varied to create a concentration gradient. The starting time for addition of an antibiotic is also varied to systematically determine any effect the biofilm may have on the development of antibiotic resistance. The antibiotic is then diluted and fresh growth media added to check for persister (dormant) cells. *P. aeruginosa* cells change their behavior when co-cultured with other cells, indicating that they detect molecules, or signaling factors, produced by other species. The response of *P. aeruginosa* to acyl homoserine lactones (AHLs) and *Pseudomonas* quinolone signal (PQS), common signaling factors for other bacterial species, is determined.

An array of nano-electrochemical sensors for providing label-free detection of redox-active small molecules and metabolites in mature biofilms is constructed. The electrochemical sensors are coupled with SERS to obtain additional information about the spatiotemporal chemical changes that take place in the biofilm. The bacteria *S. aureus* and S. *epidermidis* are used for producing biofilms as they are primarily responsible for infection in medical implants. A schematic diagram of such an array is shown in FIG. 20.

Example 18: Design and fabrication of microfluidic trapping system for bacterial cells

A two layer process is employed to create valves for directing the flow of cells and to trap them once they are near a sensor. The basic fabrication scheme is shown in FIG. 16. The fabrication process requires creating two separate, reversible master molds using photolithography. Positive photoresist (AZ4620, Clariant; FIG. 16 **1610**) is spin coated on a 75 mm diameter Pyrex wafer (ESCO Products; FIG. 16 **1620**), followed by a soft bake. The photoresist is then photolithographically patterned and developed to yield 15 $\mu$m tall raised features (**1630**). After development, the photoresist is baked for an additional 30 min to round the edges of the developed features. The patterned wafers are vapor coated with chlorotrimethylsilane (CTMS) (Dow Corning) for 75 s to facilitate release of PDMS from the master molds. Mixtures of PDMS prepolymer and curing agent (Sylgard 184, Dow Corning) are prepared in 5:1 v/v and 20:1 v/v ratios. The 5:1 PDMS mixture (20 $\mu$L) is poured on the control valve master mold (**1640**), while the 20:1 mixture is spun on the fluid channel master mold. Both pieces are then baked at 75 °C for 10 min. The PDMS layer that contains the control valve chanels (**1670**) is peeled from the master mold. The valve channels operate valves (see Fig. 17 **1780**) that control fluid flow through the microfluidic channel. Access holes are made in the control valve layer using a manual drill piece and the layer is aligned to the 20:1 fluid channel layer using a custom alignment tool. These two layers are then bonded together by baking the assembly for 30 min at 90 °C. The chip is removed from the remaining fluid channel master mold and fluid inlet/outlet holes are drilled. The chip is then placed on an unpatterned Pyrex wafer with the channels facing away from the glass and baked for an additional 2 h.

Once completed, a custom-built, chip alignment instrument is utilized to arrange the microfluidic channels (**1680**) so that they sit on top of the sensors (**1650**). Pneumatic actuation will be used to move and confine the cells in the vicinity of the sensors, thus creating a confined volume of approximately 100 picoliters. For more complicated systems, innovative designs will be required in order to efficiently transport and trap large numbers of individual cells. To simplify the number of control valves involved, a self-assembly approach may be utilized, where only a single cell is able to fit into a chamber containing sensors.

Example 19: Integration of micro and nanofluidic systems

An advantage of using nanofluidic electrochemical detection is that real-time electrochemical information can be gathered passively while permitting integration with microfluidic systems, where the technology exists for increasing the device complexity. The microfluidic sample handling and nanofluidic sensors are developed in tandem, followed by testing of the complete system. Initial electrochemical testing is conducted with known commercially available quorum sensing molecules (e.g. pyocyanin, autoinducer-2), and validation of the microfluidic architecture utilizes microscale beads to simulate bacterial cells. In the final stage of this project, bacterial cells (e.g. *E. coli, P. aeruginosa*) are transported and trapped next to the nanofluidic sensing elements using a microfluidic platform. The generation of quorum sensing molecules by the cells is monitored electrochemically while the cellular response to the molecules is observed optically. A schematic of a single bacterial cell trapped above an electrochemical sensor is shown in FIG. 17. The bacterial cell (**1790**) is suspended in a medium within a microchannel (**1770**) sealed off by valves (**1780**); the bacterium is shown bound to a recognition coating (**1760**). The redox active substance (**1720**) produced by the bacterium is detected by the electrodes (**1730** and **1740**) which form walls of the nanofluidic channel disposed within the insulating substrate (**1710**). The platform (**1760**) is separated from the upper electrode by an insulating layer (**1750**). The cells will be imaged from the top using a stereomicroscope while electrochemical information is being obtained simultaneously. Cellular activity, such as biofilm formation is correlated with the production of quorum sensing molecules.

Example 20: Detection of pyocyanin in biological fluids.

A nanoelectrode assembly was used for measuring the concentration of pyocyanin mixed into different biological fluids. Graphs of current measured for detecting pyocyanin in biological fluids using a commercial sensor Ag/AgCl reference electrode are shown in FIGS. 21 A-E. Defined amounts of pyocyanin were added to the biological fluids. The various biological fluids were bronchial lavage (A), sputum (B), urine (C), blood (D) and blood with heparin (E). FIG. 21F is a graph showing a linear relationship between the current measured and concentration of pyocyanin in different biological fluids. A summary of the results is shown in Table 1.

Table 1: Testing of pyocyanin present in biological samples

| Biological sample | Sensitivity (nA/$\mu$M) | Limit of detection ($\mu$M) | $R^2$ | Viscosity (cP) |
|---|---|---|---|---|
| Bronchial lavage | $4.63038 \times 10^{-8}$ | 13.7 | 0.994 | 0.95 |
| Urine | $2.25588 \times 10^{-7}$ | 0.814 | 0.994 | 1.087 |
| Sputum | $1.11083 \times 10^{-7}$ | 4.33 | 0.998 | 5.29 |
| Blood-no additive | $4.64911 \times 10^{-8}$ | 0.198 | 0.874 | 4.2-5.62 |
| Blood heparin | $2.06581 \times 10^{-8}$ | 1.76 | 0.991 | 4.2-5.62 |
| Lysogeny broth | $2.71869 \times 10^{-7}$ | 0.826 | 0.994 | N/A |

Example 21: Screening materials in the prevention and removal of bacterial biofilms

The properties of biofilms produced by the same species can vary dramatically depending on the external conditions to which the cells are exposed. For applications such as testing of antimicrobial materials and coatings, it is useful to determine whether or not a biofilm is forming or being removed. Microfluidic SPRI setup described herein is useful for testing various antimicrobial coatings and materials. GFP E. coli cells were grown on the prism surface inside an incubator for several hours and allowed to dry. The prism covered with the dry biofilm was placed inside an SPRI system (FIG. 18) and difference images were taken (not shown). Deionized water was run over the surface for 5.5 min. Ethanol then was flowed over the surface for 5 minutes. The images showed that there was no material left on the surface and that all bacteria had been removed. To confirm that the cleaning procedure was effective, LB growth media was spotted on the surface and the prism was placed in the incubator for 1 day. After 24 hours, the prism surface was inspected again using SPRI and no growth was detected in the media over prism surface.

The sensor surface can be modified with various chemical coatings both nonspecifically and covalently. Since the surface plasmon extends about 200 nm away from the surface, monolayer and thin coatings can be employed without sacrificing sensitivity, although the field decays exponentially with distance. The imaging capability combined with customizable microfluidic geometries is used to perform multiple tests simultaneously. The channel wall opposite the sensor surface can be modified if the modification adversely affects sensor response. Since the channels are only a few micrometers tall, it is possible to monitor biofilm formation as cells accumulate and span the height of the channel. Solutions containing diluted cell concentrations can be flowed through the channels at varying flow rates to monitor adhesion.

Reference is made to an article authored by Thaddaeus A. Webster and Edgar D. Goluch, titled "Electrochemical Detection of Pyocyanin in Nanochannels with Integrated Palladium Hydride Reference Electrodes" Lab Chip, 2012, 12, 5195-5201; and an article authored by Thaddaeus A. Webster , Hunter J. Sismaet, and Edgar D. Goluch, titled "Amperometric detection of pyocyanin in nanoftuidic channels", Nano Life 2013, 3, 1340011-1 to 1340011-8. While the present disclosure has been described in conjunction with certain preferred embodiments according to the present disclosure, one of ordinary skill, after reading the foregoing specification, will be able to effect various changes, substitutions of equivalents, and other alterations to the compositions and methods set forth herein.

As used herein, "consisting essentially of" does not exclude materials or steps that do not materially affect the basic and novel characteristics of the claim. Any recitation herein of the term "comprising", particularly in a description of components of a composition or in a description of elements of a device, can be exchanged with "consisting essentially of" or "consisting of".

While the present disclosure has been described in conjunction with certain preferred embodiments according to the present disclosure, one of ordinary skill, after reading the foregoing specification, will be able to effect various changes, substitutions of equivalents, and other alterations to the compositions and methods set forth herein.

**Claims**

1.    A device for measurement of a concentration of a redox active substance (170) in a sample, the device comprising:

a nanofluidic electrode assembly comprising a nanofluidic channel (160) disposed in a substrate, a first working electrode (120) disposed in the nanofluidic channel, and a reference electrode (140) disposed in the nanofluidic channel; and
a microfluidic channel in said substrate, the microfluidic channel in fluid communication with the nanofluidic channel;

wherein the device is capable of measuring a concentration of the redox active substance (170) present in the nanofluidic channel (160) as current flow through the first working electrode.

2. The device of claim 1 further comprising a second working electrode disposed in the nanofluidic channel; wherein the device is capable of measuring a concentration of the redox active substance present in the nanofluidic channel as current flow through the first and second working electrodes when the first working electrode is held at a potential sufficient to oxidize the redox active substance and the second working electrode is held at a potential sufficient to reduce the redox active substance.

3. The device according to any of claims 1-2, further comprising a counter electrode.

4. The device according to any of claims 2-3, wherein the first and the second working electrodes are separated by a distance of 20 - 100 nm.

5. The device according to any of claims 1-4, wherein the redox active substance is pyocyanin produced by *Pseudomonas aeruginosa.*

6. The device according to any of claims 1-5, wherein the first working electrode comprises a material selected from the group consisting of: gold, electrically conductive diamond, platinum, and glassy carbon.

7. The device according to any of claims 1-6, wherein the reference electrode comprises palladium.

8. The device according to any of claims 1-7 that is configured for implantation in a patient.

9. The device according to any of claims 1-8, wherein one or both of the reference electrode and the first working electrode form at least a portion of a wall of the nanochannel.

10. The device according to any of claims 2-9, wherein the first and second working electrodes form portions of opposite facing walls of the nanochannel.

11. The device according to any of claims 1-10, wherein the size of the nanofluidic channel precludes access of cells to the electrodes.

12. A device for simultaneous measurement of a concentration of each of a plurality of different redox active substances in a sample, the device comprising a plurality of devices according to any of claims 1-11.

13. A method of fabricating a nanoscale device according to any of claims 1-12 for measurement of a concentration of a redox active substance in a sample, the device comprising a nanofluidic electrode assembly in fluid communication with a microfluidic channel, the method comprising the steps of:

   depositing a first electrode layer on a substrate;
   depositing a sacrificial layer on the first electrode layer;
   depositing a reference electrode layer on the sacrificial layer;
   depositing an insulating layer on the reference electrode layer;
   forming two or more holes through the insulating layer, the holes providing a fluid connection to the sacrificial layer;
   applying an etching agent through the holes, whereby the sacrificial layer is etched away to form a nanochannel, the first electrode layer and reference electrode layer each forming at least a portion of a wall of the nanochannel; and
   preparing a microfluidic channel in the substrate, the microfluidic channel in fluid connection with the nanochannel.

14. A method of measuring a concentration of a redox active substance present in a sample liquid comprising:

   (i) providing a device according to any of claims 1-12 comprising the sample liquid loaded into the nanofluidic channel of the device;
   (ii) charging the reference electrode to stabilize its potential;
   (iii) recording current flow through the first working electrode; and
   (iv) determining a concentration of the substance producing the current flow by using a previously determined correlation between known concentrations of the substance and the current flow through the first working electrode recorded according to steps (i) -(iii).

15. The method of claim 14, wherein the device further comprises a second working electrode, the method comprising:

applying a potential suitable for oxidizing the substance to the first working electrode, and a potential suitable for reducing the substance to the second working electrode; and
wherein the substance has a half way potential value between the potential of the first working electrode and the potential of the second working electrode.

**Patentansprüche**

1. Vorrichtung zur Messung einer Konzentration einer redox-aktiven Substanz (170) in einer Probe, wobei die Vorrichtung Folgendes umfasst:

eine nanofluidische Elektrodenanordnung, umfassend einen nanofluidischen Kanal (160), der in einem Substrat angeordnet ist, eine erste Arbeitselektrode (120), die in dem nanofluidischen Kanal angeordnet ist, und eine Referenzelektrode (140), die in dem nanofluidischen Kanal angeordnet ist; und einen mikrofluidischen Kanal in dem Substrat, wobei der mikrofluidische Kanal in Fluidverbindung mit dem nanofluidischen Kanal steht; wobei die Vorrichtung zur Messung einer Konzentration der redox-aktiven Substanz (170), die als Stromfluss durch die erste Arbeitselektrode in dem nanofluidischen Kanal (160) vorhanden ist, in der Lage ist.

2. Vorrichtung nach Anspruch 1, ferner umfassend eine zweite Arbeitselektrode, die in dem nanofluidischen Kanal angeordnet ist;
wobei die Vorrichtung zur Messung einer Konzentration der redox-aktiven Substanz, die als Stromfluss durch die erste und zweite Arbeitselektrode in dem nanofluidischen Kanal vorhanden ist, wenn die erste Arbeitselektrode bei einem Potential gehalten wird, das ausreichend ist, um die redox-aktive Substanz zu oxidieren und die zweite Arbeitselektrode bei einem Potential gehalten wird, das ausreichend ist, um die redox-aktive Substanz zu reduzieren, in der Lage ist.

3. Vorrichtung nach einem der Ansprüche 1-2, ferner umfassend eine Gegenelektrode.

4. Vorrichtung nach einem der Ansprüche 2-3, wobei die erste und die zweite Arbeitselektrode durch einen Abstand von 20-100 nm getrennt sind.

5. Vorrichtung nach einem der Ansprüche 1-4, wobei die redox-aktive Substanz durch *Pseudomonas aeruginosa* produziertes Pyocyanin ist.

6. Vorrichtung nach einem der Ansprüche 1-5, wobei die erste Arbeitselektrode ein Material umfasst, das aus der Gruppe ausgewählt ist, die aus Folgendem besteht: Gold, elektrisch leitfähigem Diamanten, Platin und Glaskohlenstoff.

7. Vorrichtung nach einem der Ansprüche 1-6, wobei die Referenzelektrode Palladium umfasst.

8. Vorrichtung nach einem der Ansprüche 1-7, die zur Implantation in einem Patienten ausgelegt ist.

9. Vorrichtung nach einem der Ansprüche 1-8, wobei eine oder beide von der Referenzelektrode und der ersten Arbeitselektrode zumindest einen Abschnitt einer Wand des Nanokanals bilden.

10. Vorrichtung nach einem der Ansprüche 2-9, wobei die erste und zweite Arbeitselektrode Abschnitte von einander abgewandten Wänden des Nanokanals bilden.

11. Vorrichtung nach einem der Ansprüche 1-10, wobei die Größe des nanofluidischen Kanals den Zugang von Zellen zu den Elektroden ausschließt.

12. Vorrichtung zur simultanen Messung einer Konzentration von jeder aus einer Vielzahl von verschiedenen redox-aktiven Substanzen in einer Probe, wobei die Vorrichtung eine Vielzahl von Vorrichtungen nach einem der Ansprüche 1-11 umfasst.

**13.** Verfahren zur Herstellung einer Vorrichtung im Nanomaßstab nach einem der Ansprüche 1-12 zur Messung einer Konzentration einer redox-aktiven Substanz in einer Probe, wobei die Vorrichtung eine nanofluidische Elektrodenanordnung in Fluidverbindung mit einem mikrofluidischen Kanal umfasst, wobei das Verfahren die folgenden Schritte umfasst:

Abscheiden einer ersten Elektrodenschicht auf einem Substrat;
Abscheiden einer Opferschicht auf der ersten Elektrodenschicht;
Abscheiden einer Referenzelektrodenschicht auf der Opferschicht;
Abscheiden einer Isolierschicht auf der Referenzelektrodenschicht;
Bilden von zwei oder mehreren Löchern durch die Isolierschicht, wobei die Löcher eine Fluidverbindung zu der Opferschicht bereitstellen;
Auftragen eines Ätzmittels durch die Löcher, wodurch die Opferschicht weggeätzt wird, um einen Nanokanal zu bilden, wobei die erste Elektrodenschicht und die Referenzelektrodenschicht jeweils zumindest einen Abschnitt einer Wand des Nanokanals bilden; und
Herstellen eines mikrofluidischen Kanals in dem Substrat, wobei der mikrofluidische Kanal in Fluidverbindung mit dem Nanokanal ist.

**14.** Verfahren zur Messung einer Konzentration einer redox-aktiven Substanz, die in einer Probeflüssigkeit vorhanden ist, umfassend:

(i) Bereitstellen einer Vorrichtung nach einem der Ansprüche 1-12, umfassend die in den nanofluidischen Kanal der Vorrichtung geladene Probeflüssigkeit;
(ii) Laden der Referenzelektrode, um ihr Potential zu stabilisieren;
(iii) Aufzeichnen von Stromfluss durch die erste Arbeitselektrode; und
(iv) Bestimmen einer Konzentration der Substanz, die den Stromfluss erzeugt, durch Verwenden einer zuvor bestimmten Korrelation zwischen bekannten Konzentrationen der Substanz und dem gemäß Schritt (i)-(iii) aufgezeichneten Stromfluss durch die erste Arbeitselektrode.

**15.** Verfahren nach Anspruch 14, wobei die Vorrichtung ferner eine zweite Arbeitselektrode umfasst, wobei das Verfahren Folgendes umfasst:

Anwenden eines Potentials, das zum Oxidieren der Substanz geeignet ist, auf die erste Arbeitselektrode, und eines Potentials, das zum Reduzieren der Substanz geeignet ist, auf die zweite Arbeitselektrode; und wobei die Substanz einen Halbpotentialwert zwischen dem Potential der ersten Arbeitselektrode und dem Potential der zweiten Arbeitselektrode aufweist.

**Revendications**

**1.** Dispositif de mesure d'une concentration d'une substance à activité rédox (170) dans un échantillon, le dispositif comprenant :

un ensemble d'électrodes nanofluidiques comprenant un canal nanofluidique (160) disposé dans un substrat, une première électrode de travail (120) disposée dans le canal nanofluidique, et une électrode de référence (140) disposée dans le canal nanofluidique ; et un canal microfluidique dans ledit substrat, le canal microfluidique étant en communication fluidique avec le canal nanofluidique ; ledit dispositif pouvant mesurer une concentration de la substance à activité rédox (170) présente dans le canal nanofluidique (160) pendant que le courant circule dans la première électrode de travail.

**2.** Dispositif selon la revendication 1 comprenant en outre une seconde électrode de travail disposée dans le canal nanofluidique ; ledit dispositif pouvant mesurer une concentration de la substance à activité rédox présente dans le canal nanofluidique pendant que le courant circule dans les première et seconde électrodes de travail lorsque la première électrode de travail est maintenue à un potentiel suffisant pour oxyder la substance à activité rédox et la seconde électrode de travail est maintenue à un potentiel suffisant pour réduire la substance à activité rédox.

3. Dispositif selon l'une quelconque des revendications 1-2, comprenant en outre une contre-électrode.

4. Dispositif selon l'une quelconque des revendications 2-3, dans lequel les première et seconde électrodes de travail sont séparées par une distance de 20 à 100 nm.

5. Dispositif selon l'une quelconque des revendications 1-4, dans lequel la substance à activité rédox est la pyocyanine produite par *Pseudomonas aeruginosa.*

6. Dispositif selon l'une quelconque des revendications 1-5, dans lequel la première électrode de travail comprend un matériau choisi dans le groupe constitué par : l'or, le diamant électroconducteur, le platine et le graphite vitrifié.

7. Dispositif selon l'une quelconque des revendications 1-6, dans lequel l'électrode de référence comprend du palladium.

8. Dispositif selon l'une quelconque des revendications 1-7 qui est conçu pour être implanté chez un patient.

9. Dispositif selon l'une quelconque des revendications 1-8, dans lequel l'électrode de référence et/ou la première électrode de travail forment au moins une partie d'une paroi du nanocanal.

10. Dispositif selon l'une quelconque des revendications 2-9, dans lequel les première et seconde électrodes de travail forment des parties des parois en regard l'une de l'autre du nanocanal.

11. Dispositif selon l'une quelconque des revendications 1-10, dans lequel la taille du canal nanofluidique empêche les cellules d'accéder aux électrodes.

12. Dispositif de mesure simultanée d'une concentration de chacune d'une pluralité de substances à activité rédox différentes dans un échantillon, le dispositif comprenant une pluralité de dispositifs selon l'une quelconque des revendications 1-11.

13. Procédé de fabrication d'un dispositif nanométrique selon l'une quelconque des revendications 1-12 pour la mesure d'une concentration d'une substance à activité rédox dans un échantillon, le dispositif comprenant un ensemble d'électrodes nanofluidiques en communication fluidique avec un canal microfluidique, le procédé comprenant les étapes consistant à :

    déposer une première couche d'électrode sur un substrat ;
    déposer une couche sacrificielle sur la première couche d'électrode ;
    déposer une couche d'électrode de référence sur la couche sacrificielle ;
    déposer une couche isolante sur la couche d'électrode de référence ;
    former deux trous ou plus à travers la couche isolante, les trous fournissant une liaison fluidique avec la couche sacrificielle ;
    appliquer un agent d'attaque à travers les trous, moyennant quoi la couche sacrificielle est éliminée pour former un nanocanal, la première couche d'électrode et la couche d'électrode de référence formant chacune au moins une partie d'une paroi du nanocanal ; et
    préparer un canal microfluidique dans le substrat, le canal microfluidique étant en liaison fluidique avec le nanocanal.

14. Procédé de mesure d'une concentration d'une substance à activité rédox présente dans un liquide échantillon comprenant:

    (i) l'utilisation d'un dispositif selon l'une quelconque des revendications 1-12 comprenant le liquide échantillon chargé dans le canal nanofluidique du dispositif ;
    (ii) le chargement de l'électrode de référence pour stabiliser son potentiel ;
    (iii) l'enregistrement de la circulation du courant à travers la première électrode de travail ; et
    (iv) la détermination d'une concentration de la substance produisant la circulation du courant en utilisant une corrélation déterminée au préalable entre des concentrations connues de la substance et la circulation du courant à travers la première électrode de travail enregistrée selon les étapes (i)-(iii).

15. Procédé selon la revendication 14, dans lequel le dispositif comprend en outre une seconde électrode de travail, le procédé comprenant :

L'application d'un potentiel approprié pour oxyder la substance au niveau de la première électrode de travail, et un potentiel approprié pour réduire la substance au niveau de la seconde électrode de travail ; et dans lequel la substance présente une valeur médiane de potentiel entre le potentiel de la première électrode de travail et le potentiel de la seconde électrode de travail.

Au Working
Electrode

Access Holes

— 120

— 150

Pd Reference
Electrode

— 140

5μm

**FIG. 1A**

— 120
— 110

130 —

— 140
— 120

— 110

150 —

— 150

— 160

**FIG. 1B**

170

140

Pd Reference
Electrode

120

Au Working
Electrode

## FIG. 1C

## FIG. 1D

FIG. 2

**FIG. 3A**

**FIG. 3B**

Current (A)=1.49523x10$^{-10}$Pyocyanin($\mu$M)
$R^2$=0.94171

**FIG. 4**

**FIG. 5**

**FIG. 6**

**FIG. 7**

**FIG. 8A**

**FIG. 8B**

**FIG. 9**

**FIG. 10**

FIG. 11

FIG. 12A

FIG. 12B

**FIG. 13A**

**FIG. 13B**

*FIG. 14*

A)

**FIG. 15A**

B)

**FIG. 15B**

Control Layer

Fluid Layer

1620

1610

1630

1640

1670

Pyrex wafer
photoresist
PDMS
nanosensor
Silicon substrate

1680

1650

1660

**FIG. 16**

1790

1780

1770

bacteria

50 nm

1710

1720

1730

1740

1750

1760

**FIG. 17**

*FIG. 18A*

## FIG. 18B

## FIG. 18C

## FIG. 18D

## FIG. 18E

*FIG. 19*

**electrochemical sensors 2010**

**bacterial cells 2060**

**microscope slide 2050**

**2030 valve**

**2020 biofilm**

**2040 microchannel**

*FIG. 20*

FIG. 21A

FIG. 21B

FIG. 21C

**FIG. 21D**

**FIG. 21E**

F

$I=4.63036 \times 10^{-8}C$
$R^2=0.994$
$I=2.25588 \times 10^{-7}C$
$R^2=0.994$
$I=4.64911 \times 10^{-8}C$
$R^2=0.874$
$I=1.11083 \times 10^{-7}C$
$R^2=0.998$
$I=2.06581 \times 10^{-8}C$
$R^2=0.991$
$I=2.71869 \times 10^{-7}C$
$R^2=0.994$

LB

Urine

Sputum

Bronchial Lavage

Blood with Heparin

Blood

Current (μA)

Concentration (μM)

*FIG. 21F*

EP 2 875 343 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2011155586 A1

### Non-patent literature cited in the description

- **ZHANG et al.** *Curr Opin Microbial,* 2005, vol. 8, 276-281
- **JACOB et al.** *Current Opinion in Chem Biol,* 2011, vol. 15, 149-155
- **DIETRICH et al.** *Science,* 2008, vol. 321, 1203-1206
- **REN et al.** *Microfluid Nanofluid,* 2012, vol. 13, 353-381
- **PARAMYTHIOTOU et al.** *Clin Infect Dis,* 2004, vol. 38, 670-677
- **PIHEL et al.** *Anal Chem,* 1995, vol. 67, 4514-4521
- **BUCK et al.** *Anal Chem,* 2001, vol. 73, 88-97
- **HENGSTENBERG et al.** *Angew Chem Int Ed Engl,* 2001, vol. 40, 905-908
- **MÜLLER et al.** *J Neuro Meth,* 1981, vol. 4, 39-52
- **CHENG et al.** *Electrophoresis,* 2007, vol. 28, 1579-1586
- **ZOU et al.** *IEEE Sens J,* 2008, vol. 8, 527-535
- **ZEVENBERGEN et al.** *Nano Lett,* 2007, vol. 7, 384-388
- **INO et al.** *Lab Chip,* 2011, vol. 11, 385-388
- **HWANG et al.** *IEEE Sens J,* 2009, vol. 9, 609-615
- **LEWIS et al.** *Anal Chem,* 2010, vol. 82, 1659-1668
- **KWAKYE et al.** *Biosens Bioelectron,* 2006, vol. 21, 2217-2223
- **SWENSEN et al.** *J Am Chem Soc,* 2009, vol. 131, 4262-4266
- **WANG et al.** *Sensors,* 2008, vol. 8, 2043-2081
- **STRAVER et al.** *Lab Chip,* 2012, vol. 12, 1548-1553
- **DAM et al.** *Analyst,* 2007, vol. 132, 365-370
- **NIWA et al.** *Electroanal,* 1991, vol. 3, 163-168
- **GOLUCH et al.** *Anal Bioanal Chem,* 2009, vol. 394, 447-456
- **KÄTELHÖN et al.** *Anal Chem,* 2010, vol. 82, 8502-8509
- **WEBSTER T A.** Detection of quorum sensing molecules in nanofluidic detection devices. NORTHEASTERN UNIVERSITY, 23 July 2011
- **LACHER et al.** *Electrophoresis,* 2001, vol. 22, 2526-2536
- **STRAVER et al.** *Lab Chip,* vol. 12, 1548-1553
- **ZEVENBERGEN et al.** *Nano Lett,* 2011, vol. 11, 2881-2886
- **WOLFRUM et al.** *Anal Chem,* 2008, vol. 80, 972-977
- **WEBSTER ; GOLUCH.** *Lab Chip,* 2012, vol. 12, 5195-5201
- **CZERWINSKI et al.** *J Electroanal Chem Interfacial Electrochem,* 1991, vol. 316, 211-221
- **CZERWINSKI ; MARASSI.** *J Electroanaly Chem Interfacial Electrochem,* 1992, vol. 322, 373-381
- **CZERWINSKI et al.** *J Electroanal Chem Interfacial Electrochem,* 1995, vol. 386, 207-211
- **ZENG et al.** *Nano Lett,* 2011, vol. 11, 262-268
- **ODIJK et al.** *Lab Chip,* 2009, vol. 9, 1687-1693
- **SHARP et al.** *Bioelectrochem,* 2010, vol. 77, 114-119
- **IMOKAWA et al.** *Anal Chem,* 2006, vol. 78, 265-271
- **SILVA et al.** *J Brazil Chem Soc,* 2009, vol. 20, 1235-1241
- **CHUNG-CHIUN.** *Appl Biochem Biotech,* 1993, vol. 41, 99-107
- **WANG ; NEWMAN.** *Environ Sci Tech,* 2008, vol. 42, 2380-2386
- **WEBSTER ; E. D. GOLUCH.** *Lab Chip,* 2012, vol. 12, 5195-5201
- **ODIJK et al.** *Electroanal,* 2008, vol. 20, 463-468
- **ABADIAN et al.** Real-time detection of bacterial biofilm growth using surface plasmon resonance imaging. *microTAS,* 2012, 413-415
- **YANASE et al.** *Biosensors and Bioelectronics,* 2010, vol. 26 (2), 674-81
- **THADDAEUS A. WEBSTER ; EDGAR D. GOLUCH.** Electrochemical Detection of Pyocyanin in Nanochannels with Integrated Palladium Hydride Reference Electrodes. *Lab Chip,* 2012, vol. 12, 5195-5201

• **THADDAEUS A. WEBSTER ; HUNTER J. SISMAET ; EDGAR D. GOLUCH.** Amperometric detection of pyocyanin in nanoftuidic channels. *Nano Life,* 2013, vol. 3, 13400-1, 1340011-8

• **THADDAEUS A. WEBSTER ; HUNTER J. SISMAET ; EDGAR D. GOLUCH.** Amperometric detection of pyocyanin in nanoftuidic channels. *Nano Life,* 2013, vol. 3, 13400-1, 1340011-8